# EUROPEAN PATENT APPLICATION

(11) **EP 2 543 720 A1**
(43) Date of publication of application: **09.01.2013**
(21) Application number: 11173033.9
(22) Date of filing: 07.07.2011
(51) Int. Cl.: C12N 7/00, C12N 15/70

(54) **Genetically modified phage and use thereof**

(71) Applicant: Delphi Genetics, 6041 Charleroi (BE)
(72) Inventor: Szpirer, Cédric, 6224 Fleurus (BE)
(74) Representative: Icosa

(57) **Abstract**

The present invention relates to a genetically modified phage and use thereof in a method for producing a biomolecule of interest.

## Description

### FIELD OF INVENTION

The present invention relates to the field of production of biomolecules of interest in biologic systems. Specifically, the present invention relates to a genetically modified phage, and use thereof in order to avoid phage contamination of the culture broth and/or bacterial lysis.

### BACKGROUND OF INVENTION

Bacterial cells are systems of choice for the production of biomolecules, especially of target proteins. Among other advantages, bacterial systems are easy to use and allow the rapid production of large quantities of proteins in a limited volume of culture.

One of the most widely and routinely used bacterial system is the bacteriophage T7 expression system. This bacterial system was described in US 4,952,496. In this system, the gene encoding the target protein is placed under the control of a T7 promoter, and is transformed in a bacterial host, usually *E. coli,* which comprises an integrated lambda DE3 lysogen phage. The lambda DE3 lysogen phage carries the gene encoding the T7 RNA polymerase under the control of a *lacUV5* promoter. When cultured on an IPTG-containing medium, the expression of the T7 RNA polymerase is induced, and allows the expression of the target protein.

Due to the integration of the gene of the T7 RNA polymerase (*T7 gene 1*) within the sequence of the *Int* gene, Lambda DE3 phage should be defective in its ability to enter into the lytic phase. However, bacterial lysis is observed during some protein productions and in the absence of any other phage, suggesting that the DE3 phage may recover its lytic properties. The bacterial lysis and even more, the presence of infectious phages in the culture broth is highly problematic because (i) it compromises the use of produced target proteins for some applications, such as, for example, pharmaceutical applications, (ii) the decontamination process in order to remove any trace of phages requires the shutdown of the production lines and the complete renewal of the batches of culture and (iii) it reduces dramatically the yield of recombinant protein.

Alternative methods to the use of a phage have been described:
WO03/050240 describes an expression system for producing a target protein in a host cell comprising a gene encoding T7 RNA polymerase integrated using homologous recombination. However, the system of WO03/050240 is difficult to implement, due to the size of the T7 RNA polymerase gene and due to the fact that homologous recombination is not possible or easy to do in all E. coli strains (as mentioned by Phue et al., Biotechnology and Bioengineering, 101, 831-836, 2008). Consequently, the number of transformed cells carrying the T7 RNA polymerase integration remains very low or these cells are not obtained. Moreover, using homologous recombination, it is necessary to use a selective marker to select bacteria containing integration of the T7 RNA polymerase gene. This marker will not be usable for another selection step and could be undesired for the final use of the strain. For example, selective markers often used are antibiotic resistance genes but it is recommended to avoid these genes in biopharmaceutical productions. An additional step is thus required to remove the antibiotic resistance gene from the strain and it is not always possible to do it.

WO2008/139153 describes another expression system, wherein the host cell is transformed with a plasmid comprising an expression cassette for T7 RNA polymerase. However, due to the use of a plasmid, hosts cells have to be maintained in selective conditions to make sure that they still comprise the plasmid. In addition, plasmids are frequently subjected to recombination, which impaired the expression system.

Therefore, there is a need for a novel method for producing a biomolecule of interest, wherein, when a phage is used, the culture is not contaminated by infectious phages or unintentionally lysed during growth or protein production.

The present invention hereby provides a genetically modified phage that does not recover its lytic properties during culture, thereby allowing the production of a biomolecule of interest without phage contamination.

### SUMMARY

One object of the invention is a genetically modified phage wherein
- an expression system is inserted,
- the *S* and/or the *R* genes are inactivated.

In one embodiment of the invention, the *Int* and/or *Xis* gene is inactivated.

In another embodiment of the invention, the *Rz* gene is inactivated.

In another embodiment of the invention, an expression system is inserted and the *Int* gene, the *Xis* gene and the *R*, *S* and *Rz* genes are inactivated.

In another embodiment of the invention, the expression system is the T7 expression system.

In another embodiment of the invention, the phage is the lambda phage, the 434phage, the phi80 phage, the phi81 phage, the HK97 phage, the P21 phage.

In another embodiment of the invention, the genetically modified phage as described here above has the sequence SEQ ID NO: 10.

Another object of the invention is a kit comprising the genetically modified phage as described here above and a helper phage.

Another object if the invention is a host cell comprising the genetically modified phage as described here above.

In one embodiment of the invention, the host cell is an enterobacteria, preferably E. Coli.

In another embodiment of the invention, the host cell as described here above further comprises the inactivation of at least one of the genes *tonA, galK, araB, araA, lon, ompT, rcsA, hsdR, mrr, endA* and *recA.*

In another embodiment of the invention, the host cell as described here above comprises the insertion of the ccdb gene.

Another object of the invention is a kit comprising the host cell as described here above and a plasmid comprising the ccdA gene.

Another object of the invention is a process for preparing a host cell as described here above, comprising infecting a host cell with a genetically modified phage as described here above.

Another object of the invention is a process for producing a biomolecule of interest, comprising
- cultivating a host cell comprising the genetically modified phage according to any one of claims 1 to 7 and the nucleic acid sequence of the biomolecule of interest,
- recovering the biomolecule of interest.

### DETAILED DESCRIPTION

The present invention relates to a genetically modified phage, wherein the ability of the phage to regain its lytic properties is limited.

The Inventors focused on the genetic modification of a phage. Surprisingly, the Inventors showed that it was not possible to delete all viral sequences of the integrated phage, because the viability of the infected bacteria was severely compromised in that case. In particular, the Inventors showed that the deletion of a DNA fragment comprising the coding sequences of the *ral* gene and the *N* gene leads to the death of the host cell (See EXAMPLES). This result was surprising because the *ral* and *N* genes are not known to be involved in the lysogenic state; the *N* gene is only described as essential for lytic growth. On the contrary, in lysogenic state, a repressor of the phage, named CI or C2, blocks the expression of N and ral.

The present invention thus relates to a genetically modified phage wherein
- an expression system is inserted, and
- at least one of the *Int*, *Xis*, *R*, *S* and *Rz* genes are inactivated.

In one embodiment, the genetically modified phage comprises an expression system and the *S* gene is inactivated.

In one embodiment, the genetically modified phage comprises an expression system and the *R* gene is inactivated.

In one embodiment, the genetically modified phage comprises an expression system and the *Int* gene is inactivated.

In one embodiment, the genetically modified phage comprises an expression system and the *Xis* gene is inactivated.

In one embodiment, the genetically modified phage comprises an expression system and the *S* and *Int* genes are inactivated.

In one embodiment, the genetically modified phage comprises an expression system and the *S* and *Xis* genes are inactivated.

In one embodiment, the genetically modified phage comprises an expression system and the *S* and *R* genes are inactivated.

In one embodiment, the genetically modified phage comprises an expression system and the *S* and *Rz* genes are inactivated.

In one embodiment, the genetically modified phage comprises an expression system and the *R* and *Rz* genes are inactivated.

In one embodiment, the genetically modified phage comprises an expression system and the *R* and *Int* genes are inactivated.

In one embodiment, the genetically modified phage comprises an expression system and the *R* and *Xis* genes are inactivated.

In one embodiment, the genetically modified phage comprises an expression system and the *S*, *Int* and *Xis* genes are inactivated.

In one embodiment, the genetically modified phage comprises an expression system and the *S*, *Int* and *Rz* genes are inactivated.

In one embodiment, the genetically modified phage comprises an expression system and the *S*, *Int* and *R* genes are inactivated.

In one embodiment, the genetically modified phage comprises an expression system and the *S*, *Xis* and *Rz* genes are inactivated.

In one embodiment, the genetically modified phage comprises an expression system and the *S*, *Xis* and *R* genes are inactivated.

In one embodiment, the genetically modified phage comprises an expression system and the *S*, *R* and *Rz* genes are inactivated.

In one embodiment, the genetically modified phage comprises an expression system and the *R, Int* and *Xis* genes are inactivated.

In one embodiment, the genetically modified phage comprises an expression system and the *R, Int* and *Rz* genes are inactivated.

In one embodiment, the genetically modified phage comprises an expression system and the *R, Xis* and *Rz* genes are inactivated.

In one embodiment, the genetically modified phage comprises an expression system and the *S, Int, Xis* and *Rz* genes are inactivated.

In one embodiment, the genetically modified phage comprises an expression system and the *S, Int, Xis* and *R* genes are inactivated.

In one embodiment, the genetically modified phage comprises an expression system and the *S, Int, R* and *Rz* genes are inactivated.

In one embodiment, the genetically modified phage comprises an expression system and the *S, R, Xis* and *Rz* genes are inactivated.

In one embodiment, the genetically modified phage comprises an expression system and the *R, Rz, Xis* and *Int* genes are inactivated.

In one embodiment, the genetically modified phage comprises an expression system and the *S, R, Rz, Xis* and *Int* genes are inactivated.

Examples of phages which can be used in the invention include, but are not limited to, the lambda (λ) phage, lambda-like and lambdoid phages. Lambda phage, also known as coliphage lambda, is a virus that infects Escherichia coli. Lambda is a temperate bacteriophage. Lambda-like phages form a family of bacteriophages and archaeal viruses which are characterized by long, non-contractile tails. Lambdoid phages are natural relatives of lambda phage. Most of them grow on E. coli, but a few come from other host cells, such as, for example, Salmonella typhimurium. These phages may have the same gene order as lambda.

Examples of lambda-like and lambdoid phages which could be used in the present invention include, but are not limited to, coliphage 434, phi80, phi81, HK97, P21 and P22.

In an embodiment, the phage is lambda (Enterobacteria phage lambda, accession number NC_001416). The organization of the genome of the lambda phage is shown in table 1 below.

**Table 1**

| **Start** | **End** | **Name** | **Description** |
|---|---|---|---|
| 191 | 736 | nul | DNA packaging protein |
| 711 | 2636 | A | DNA packaging protein |
| 2633 | 2839 | W | head-tail joining protein |
| 2836 | 4437 | B | capsid component |
| 4418 | 5737 | C | capsid component |
| 5132 | 5737 | nu3 | capsid assembly protein |
| 5747 | 6079 | D | head-DNA stabilization protein |
| 6135 | 7160 | E | capsid component |
| 7202 | 7600 | Fi | DNA packaging protein |
| 7612 | 7965 | Fii | head-tail joining protein |
| 7977 | 8555 | Z | tail component |
| 8552 | 8947 | U | tail component |
| 8955 | 9695 | V | tail component |
| 9711 | 10133 | G | tail component |
| 10115 | 10549 | T | tail component |
| 10542 | 13103 | H | tail component |
| 13100 | 13429 | M | tail component |
| 13429 | 14127 | L | tail component |
| 14276 | 14875 | K | tail component |
| 14773 | 15444 | I | tail component |
| 15505 | 18903 | J | tail:host specificity protein |
| 18965 | 19585 | lom | outer host membrane |
| 19650 | 20855 | orf-401 | Tail fiber protein |
| 20767 | 20147 | orf206b | hypothetical protein |
| 21029 | 21973 | orf-314 | Tail fiber |
| 21973 | 22557 | orf-194 | Putative fiber assembly protein |
| 23918 | 22686 | ea47 | ea47 |
| 25399 | 24509 | ea31 | ea31 |
| 26973 | 25396 | ea59 | ea59 |
| 28882 | 27812 | int | integration protein |
| 29078 | 28860 | xis | Excisionase |
| 29285 | 29118 | hypothetical | hypothetical protein |
| 29655 | 29374 | ea8.5 | ea8.5 |
| 30395 | 39847 | ea22 | ea22 |
| 31024 | 30839 | orf61 | hypothetical protein |
| 31196 | 31005 | orf63 | hypothetical protein |
| 31351 | 31169 | orf60a | hypothetical protein |
| 32028 | 31348 | exo | exonuclease |
| 32810 | 32025 | bet | bet |
| 33232 | 32816 | gam | host-nuclease inhibitor protein Gam |
| 33330 | 33187 | kil | host-killing protein |
| 33463 | 33299 | cIII | antitermination protein |
| 35582 | 33494 | ea10 | Putative single-stranded DNA binding protein |
| 35582 | 33930 | ral | restriction alleviation protein |
| 34357 | 34271 | orf28 | hypothetical protein |
| 34482 | 35036 | lambdap48 | Superinfection exclusion protein B |
| 35582 | 34560 | N | early gene regulator |
| 36259 | 35825 | rexb | exclusion protein |
| 37114 | 36275 | rexa | exclusion protein |
| 37940 | 37227 | cI | repressor |
| 38023 | 38135 | cro | antirepressor |
| 38360 | 38653 | cII | transcriptional activator |
| 38686 | 39585 | O | DNA replication protein |
| 39582 | 40283 | P | DNA replication protein |
| 40280 | 40570 | ren | ren exclusion protein |
| 40644 | 41084 | NinB | NinB |
| 41081 | 41953 | NinC | NinC protein |
| 41950 | 42123 | NinD | NinD protein |
| 42090 | 42272 | NinE | NinE protein |
| 42269 | 42439 | NinF | NinF protein |
| 42429 | 43043 | NinG | NinG protein |
| 43040 | 43246 | NinH | NinH protein |
| 43224 | 43889 | NinI | NinI protein |
| 43886 | 44509 | Q | late gene regulator |
| 44621 | 44815 | orf-64 | hypothetical protein |
| 45186 | 45509 | S | Cell lysis protein |
| 45493 | 45969 | R | endolysin |
| 45966 | 46427 | Rz | cell lysis protein |
| 46186 | 46368 | Rz1 | Rz1 protein |
| 46752 | 46459 | bor | Bor protein precursor |
| 47575 | 47042 | lambdap78 | putative enveloppe protein |
| 47738 | 47944 | lambdap79 | hypothetical protein |

In the present invention, the position of the residues within the sequence of the lambda phage relates to NC_001416.

In another embodiment, the phage is lambda DE3 (accession number EU078592). The Lambda DE3 phage is a modified lambda phage D69, comprising the gene encoding the T7 RNA polymerase under the control of a lacUV5 promoter. The list of the genes carried by the sequence of Lambda DE3 and their position are shown in the Table 2 below.

**Table 2**

| **Start** | **End** | **Name** | **Description** |
|---|---|---|---|
| 341 | 1423 | lacI | lactose operon repressor |
| 1546 | 1995 | lacZ | N-terminal fragment of beta-galactosidase |
| 2026 | 4677 | 1 | T7 DNA-directed RNA polymerase |
| 5804 | 5586 | xis | excisionase |
| 6011 | 5844 | hypothetical | Hypothetical protein |
| 6381 | 6100 | ea8.5 | ea8.5 |
| 7121 | 6573 | ea22 | ea22 |
| 7750 | 7565 | hypothetical | Hypothetical protein |
| 7922 | 7731 | hypothetical | Hypothetical protein |
| 8077 | 7895 | hypothetical | Hypothetical protein |
| 8754 | 8074 | exo | exonuclease |
| 9536 | 8751 | bet | Bet |
| 9958 | 9542 | gam | host-nuclease inhibitor protein Gam |
| 10056 | 9913 | kil | host-killing protein |
| 10189 | 10025 | cIII | antitermination protein |
| 10630 | 10262 | ea10 | putative single-stranded DNA binding protein |
| 11013 | 10813 | ral | restriction alleviation protein |
| 11083 | 10997 | hypothetical | Hypothetical protein |
| 11391 | 11092 | N | probable regulatory protein N (early gene regulator) |
| 12356 | 11706 | C2 | repressor protein C2 |
| 12437 | 12622 | cro | regulatory protein cro (Antirepressor) |
| 12738 | 13037 | cII | antitermination protein |
| 13070 | 13969 | O | DNA replication protein |
| 13966 | 14667 | P | DNA replication protein |
| 14664 | 15467 | ren | Ren exclusion protein |
| 15464 | 16087 | Q | late gene regulator |
| 16199 | 16393 | hypothetical | Hypothetical protein |
| 16764 | 17087 | S | cell lysis protein |
| 17071 | 17547 | R | cell lysis protein |
| 17544 | 18005 | Rz | cell lysis protein |
| 18330 | 18037 | Bor | Bor protein precursor |
| 19153 | 18620 | putative | putative envelope protein |
| 19316 | 19522 | hypothetical | Hypothetical protein |
| 20270 | 20815 | nul | DNA packaging protein |
| 20790 | 22715 | A | DNA packaging protein |
| 22712 | 22918 | W | head-tail joining protein |
| 22915 | 24516 | B | capsid component |
| 24497 | 25816 | C | capsid component |
| 25826 | 26158 | D | head-DNA stabilization protein |
| 26214 | 27239 | E | capsid component |
| 27281 | 27679 | Fi | DNA packaging protein |
| 27691 | 28044 | Fii | head-tail joining protein |
| 28056 | 28634 | Z | tail component |
| 28631 | 29026 | U | tail component |
| 29034 | 29774 | V | tail component |
| 29790 | 30212 | G | tail component |
| 30194 | 30628 | T | tail component |
| 30621 | 33182 | H | tail component |
| 33179 | 33508 | M | tail component |
| 33508 | 34206 | L | tail component |
| 34356 | 34955 | K | tail component |
| 34853 | 35524 | I | tail component |
| 35585 | 38983 | J | tail:host specificity protein |
| 39045 | 39665 | lom | outer host membrane |
| 39730 | 40935 | tail | tail fiber protein |
| 41109 | 41372 | tail | tail fiber |
| 42175 | 41237 | ea59 | ea59 |

In the present invention, the position of the residues within the sequence of the lambda DE3 phage relates to EU078592.

As used herein, an "expression system" refers to a linear or a circular DNA molecule composed of a fragment encoding a nucleic acid sequence operably linked to an additional fragment for the transcription of the system.

The additional fragment includes a promoter and a stop codon sequence. The expression system may further contain one or more origins of replication, one or more selection markers and a sequence encoding a ribosome binding site.

"Operably linked" means that fragments are arranged to be functioning as they are supposed to be, for example once transcription starts at the promoter, it goes through coded fragment to stop codon.

"Promoter" in the meaning of the present invention is an expression control element that permits binding of RNA polymerase and the initiation of transcription.

In one embodiment of the invention, the nucleic acid sequence is under the control of a "strong" promoter. A strong promoter is characterized by a high binding affinity of the promoter sequence to an RNA polymerase, usually the naturally occurring corresponding RNA polymerase, on the one hand and the rate of formation of mRNA by that RNA polymerase on the other hand.

In a preferred embodiment, the nucleic acid sequence is under the control of an "inducible promoter". An "inducible promoter" is a promoter that may be regulated by external factors, e.g. the presence of an inductor (also termed "inducer") molecule or the absence of a repressor molecule, or physical factors like increased or decreased temperature, osmolarity, or pH value. Different promoters and the respective induction principles were reviewed by Makrides et al. (Microbiological Reviews, 1996, (60)3: 512-538). Examples of inducible promoters which may be used in the present invention include, but are not limited to the tac or the trc promoter, the lac or the lacUV5 promoter (all inducible by lactose or its analog IPTG (isopropylthiol-ß-D-galactoside)), the tightly regulatable araBAD promoter (PBAD; Guzman et al., 1995, inducible by arabinose), the trp promoter (inducible by ß-indole acrylic acid addition or tryptophan starvation, repressible by tryptophan addition), the lambda promoter pL (λ) (induction by an increase of temperature), the phoA promoter (inducible by phosphate starvation), the PprpB (induction with propionate) or other promoters suitable for recombinant protein expression, which all use *E*. *coli* RNA polymerase.

Among inducible promoters are those that show a "leaky" expression behavior. Such promoters (so-called "leaky promoters") are, in principle, inducible, but show nevertheless also basal expression without being externally induced. Inducible promoters that show leaky expression under non-induced conditions may behave similarly to constitutive promoters (i.e. they are steadily and continuously active or they may be activated or enhanced as a result of certain cultivation conditions). Leaky promoters may be particularly useful for continuously operated cultivation processes. Examples of leaky promoters are the T7 promoter and the trp promoter.

In one embodiment of the invention, the promoter may also be constitutive, i.e. a promoter which controls expression without the need for induction on the one hand, or the possibility of repression on the other hand. Hence, there is continuous and steady expression at a certain level. As an example, the strong constitutive HCD promoter (Poo et al., Biotechnology Letters, 2002, 24:1185-1189; Jeong et al., Protein expression and purification, 2004, 36:150-156) may be applied for constitutive expression.

In one embodiment, the expression system comprises a nucleic acid sequence encoding a protein that induces the expression of the biomolecule of interest. Advantageously, the expression of the biomolecule of interest is induced in particular conditions, such as, for example, under selection.

Examples of such nucleic acid sequences include, but are not limited to, the gene encoding the T7 RNA polymerase, *T7 gene 1*. In that case, the expression of the T7 RNA polymerase induces the expression of the biomolecule of interest placed under the control of a T7 promoter.

Preferably, the expression system is the T7 expression system. The T7 expression system was described in US4,952,496, which is incorporated herein by reference. The T7 expression system comprises a DNA fragment from the T7 phage, containing the entire coding sequence for the T7 RNA polymerase (i.e. the *T7 gene 1*). Any natural active promoter of the *T7 gene 1* was removed and an inducible lacUV5 promoter was inserted ahead of the coding sequence. The lacUV5 promoter is induced by addition of IPTG to the culture medium.

According to another embodiment, the expression system comprises the nucleic acid sequence of the biomolecule of interest.

With regard to the biomolecule of interest, there are no limitations. It may, in principal, be any amino acid sequences, nucleic acid sequences, such as, for example, DNA or RNA.

Examples of amino acid sequences include polypeptide, protein or peptide that is to be produced on a manufacturing scale, e.g. an industrial biomolecule or a therapeutic biomolecule.

Examples for biomolecules that can be produced by the method of the invention are, without limitation, enzymes, regulatory proteins, receptors, peptides, e.g. peptide hormones, cytokines, membrane or transport proteins.

The biomolecules of interest may also be antigens as used for vaccination, vaccines, antigen-binding proteins, immune stimulatory proteins, allergens, full-length antibodies or antibody fragments or derivatives. Antibody derivatives may be selected from the group of single chain antibodies, (scFv), Fab fragments, Fv fragments, single domain antibodies (VH or VL fragment), domain antibodies like camelid single domain antibodies (VHH, nanobodies) or other antibody formats as described for instance in Andersen and Reilly (Current Opinion in Biotechnology, 2004, 15:456-462) or Holliger and Hudson (Nature Biotechnology, 2005 (23)9: 1126-1136).

The biomolecules of interest in the present invention can also be exemplified by protein (viral antigen), e.g., coat protein, core protein, protease, reverse transcriptase, integrase, and so forth, encoded in the genome of a pathogenic virus, e.g., hepatitis B virus, hepatitis C virus, I-HV, influenza, and so forth; growth factors such as platelet-derived growth factor (PDGF), stem cell growth factor (SCF), hepatocyte growth factor (HGF), transforming growth factor (TGF), nerve growth factor (NGF), epidermal growth factor (EGF), fibroblast growth factor (FGF), insulin-like growth factor (IGF), and so forth; cytokines such as tumor necrosis factor, interferon, interleukin, and so forth; hematopoietic factors such as erythropoietin, granulocyte colony-stimulating factor, granulocyte-macrophage colony-stimulating factor, macrophage colony-stimulating factor, thrombopoietin, and so forth; peptide hormones such as luteinizing hormone-releasing hormone (LB-RH), thyrotropin-releasing hormone (TRH), insulin, somatostatin, growth hormone, prolactin, adrenocorticotropic hormone (ACTH), melanocyte-stimulating hormone (MSH), thyroidstimulating hormone (TSH), luteinizing hormone (LU), follicle-stimulating hormone (FSH), vasopressin, oxytoxin, calcitonin, parathyroid hormone (PTH), glucagon, gastrin, secretin, pancreozymin, cholecystokinin, angiotensin, human placenta lacto8en, human chorionic gonadotropin (HCG), cerulein, motilin, and so forth; analgesic peptides such as enkephalin, endorphin, dynorphin, kyotorphin, and so forth; enzymes such as superoxide dismutase (SOD), urokinase, tissue plasminogen activator (TPA), asparaginase, kallikrein, and so forth; peptide neurotransmitters such as bombesin, neutrotensin, bradykinin, substance P, Alzheimer's amyloid peptide (AD), SOD1, presenillin 1 and 2, renin, Dsynuclein, amyloid A, amyloid P, activin, anti-HER-2, bombesin, enkephalinase, protease inhibitors, therapeutic enzymes, D 1- antitrypsin, mammalian trypsin inhibitor, mammalian pancreatic trypsin inhibitor, calcitonin, cardiac hypertrophy factor, cardiotrophins (such as cardiotrophin-1), CD proteins (such as CD-3, CD-4, CD-8 and CD-19), CFTR, CTNF, DNase, human chorionic gonadotropin, mouse gonadotropin-associated peptide, cytokines, transthyretin, amylin, lipoproteins, lymphokines, lysozyme, a growth hormone (including human growth hormone), bovine growth hormone, growth hormone releasing factor, parathyroid hormone, thyroid stimulating hormone, growth factors, brain-derived neurotrophic growth factor, epidermal growth factor (EGF), fibroblast growth factor (such as D FGF and D FGF), insulin-like growth factor-I and -II, des(1-3)-IGF-I (brain IGF-I), insulin-like growth factor binding proteins, nerve growth factor (such as NGF- D), platelet derived growth factor (PDGF), vascular endothelial growth factor (VEGF), receptors for growth hormones or growth factors, transforming growth factor (TGF) (such as TGF-D, TGF-D 1, TGF-D2, TGF-D3, TGF-D4 or TGF-D5), neurotrophic factors (such as neurotrophin-3, -4 ,-5, or -6), gelsolin, glucagon, kallikreins, mullerian- inhibiting substance, neurotrophic factors, p53, protein A or D, prorelaxin, relaxin A-chain, relaxin B-chain, rheumatoid factors, rhodopsin, a serum albumin (such as human serum albumin), inhibin, insulin, insulin chains, insulin A-chain, insulin D -chain, insulin receptor, proinsulin, luteinizing hormone, integrin, interleukins (ILs) (such as IL-1 to IL-10, IL-12, IL-13), erythropoietin, thrombopoietin, fibrillin, follicle stimulating hormone, clotting factors (such as factor VIIIC, factor IX, tissue factor, and von Willebrands factor), anticlotting factors (such as Protein C, atrial naturietic factor, lung surfactant), a plasminogen activator (such as human tissue plasminogen activator or urokinase), thrombin, tumor necrosis factor- D or D, D-ketoacid dehydrogenase, addressins, bone morphogenetic proteins (BMPs), collagen, colony stimulating factors (CSFs) (such as M-CSF, GM-CSF and G-CSF), decay accelerating factor, homing receptors, interferons (such as interferon-alpha, -gamma and -beta), keratin, osteoinductive factors, PRNP, regulatory proteins, superoxide dismutase, surface membrane proteins, transport proteins, T- cell receptors, antigens such as gpl 20(HIb) immuno toxins, atrial natriuretic peptide, seminal vesicle exocrine protein, D 2-microglobulin, PrP, precalcitonin, ataxin 1, ataxin 2, ataxin 3, ataxin 6, ataxin 7, huntingtin, androgen receptor, CREB-binding protein, gpl 20, p300, CREB, API, ras, NFAT, jun, fos, dentaorubral pallidoluysian atrophy-associated protein, a microbial protein (e.g., maltose binding protein, ABC transporter, glutathione S transferase, thioredoxin, D -lactamase), green fluorescent protein, red fluorescent protein, an enzyme such as superoxide dismu-tase, asparaginase, arginase, arginine deaminase, adenosine deaminase, ribonuclease, catalase, uricase, bilirubin oxidase, trypsin, papain, alkaline phosphatase, beta-glucoronidase, purine nucleoside phosphorylase or batroxobin, an opioid, e.g. endorphins, enkephalins or non-natural opioids, a hormone or neuropeptide, e.g. calcitonin, glucagon, gastrins, adreno- corticotropic hormone (ACTH), cholecystokinins, lutenizing hormone, gonadotropin- releassing hormone, chorionic gonadotropin, corticotrophin-releasing factor, vasopressin, oxytocin, antidiuretic hormones, thyroid-stimulating hormone, thyrotropin-releasing hormone, relaxin, prolactin, peptide YY, neuropeptide Y, pancreastic polypeptide, leptin, CART (cocaine and amphetamine regulated transcript), a CART related peptide, perilipin, melano- cortins (melanocyte-stimulating hormones) such as MC-4, melanin-concentrating hormones, natriuretic peptides, adrenomedullin, endothelin, secretin, amylin, vasoactive intestinal peptide (VIP), pituary adenylate cyclase activating polypeptide (PACAP), bombesin, bombesin- like peptides, thymosin, heparin-binding protein, soluble CD4, hypothalamic releasing facto- rand melanotonins or functional analogs thereof. In another embodiment of the invention the target protein may be a processing enzyme such as proteases (eg enterokinase, caspases trypsine like serine proteases), lipase, phospatase, glycosyl hydrolases (eg. mannosidases, xylosidases, fucosidases), kinase, mono or dioxidase, peroxidase, transglutaminase, carboxypeptidase, amidase, esterase, and phosphatase...

Preferred sources for such mammalian polypeptides include human, bovine, equine, porcine, lupine and rodent sources, with human proteins being particularly preferred. The biomolecule of interest of the present invention also encompasses variants of the aforementioned protein. These variants encompass, for example, protein that has the same activity as the aforementioned protein and that comprises an amino acid sequence with, in the amino acid sequence of the aforementioned protein, one or more deleted, substituted, inserted and/or added amino acids. Such protein can be exemplified by protein that has the same activity as the aforementioned protein and that comprises an amino acid sequence with, in the amino acid sequence of the aforementioned protein, one or more deleted, substituted, inserted and/or added amino acids. Two or more different types of modifications selected from deletion, substitution, insertion, and addition may be carried out concurrently.

The biomolecule of interest of the present invention also encompasses "partial peptides" of the aforementioned protein. A partial peptide of the protein can be exemplified by a partial peptide comprising an amino acid sequence in which a portion of the amino acid sequence of the aforementioned protein runs uninterrupted, wherein the partial peptide preferably has the same activity as said protein. Such a partial peptide can be exemplified by a polypeptide that has an amino acid sequence comprising at least 20 and preferably at least 50 of the amino acid residues in the amino acid sequence of the aforementioned protein. This polypeptide preferably contains the amino acid sequence that corresponds to the region that is involved with the activity of the aforementioned protein. In addition, the partial peptide used in the present invention may also be a partial peptide as yielded by a modification of this polypeptide wherein 1 or a plurality of amino acid residues (for example, approximately 1 to 20, more preferably approximately 1 to 10, and even more preferably approximately 1 to 5) is deleted from, substituted in, inserted into, and/or added to its amino acid sequence. The partial peptide used in the present invention can also be used as an antigen for antibody production.

In one embodiment of the invention, the biomolecule of interest is selected from the group comprising Human growth hormone, human insulin, follicle-stimulating hormone, Factor VIII, Erythropoeietin, Granulocyte colony-stimulating factor, Alpha-glactosidase A, Alpha-L-iduronidase, N-actetylgalactosamine-4-sulfatase, Dornase alfa, Tisssue plasminogen activator, Glucocerebrosidase, Interferon, Insulin-like growth factor 1, bovine somatotropin, Porcine somatotropin, bovine chymosin, and envelop protein of the hepaptitis B virus.

The biomolecule of interest also encompasses modified polypeptides or proteins that have underwent posttranslational and post-export modifications in the periplasm such as cyclization, glycosylation, phophorylation, methylation, oxidation, dehydratation, proteolytic cleavage.

In one embodiment, the biomolecule of interest is an enzyme for metabolizing a biomolecule in the extracellular medium (herein referred as "extracellular biomolecule"). In one embodiment, the extracellular biomolecule comprises a polysaccharide or a lipid. In one embodiment of the invention, the polysaccharide comprises alginate, pectin, cellulose, cellobiose, laminarin, or a mixture thereof. In one embodiment of the invention, the lipid comprises a fatty acid, a glycolipid, a betaine lipid, a glycerolipid, a phospholipid, a glycerolphospholipid, a sphingolipid, a sterol lipid, a prenol lipid, a saccharolipid, a polyketide, or a mixture thereof. In one embodiment of the invention, the biomolecule of interest is an enzyme converting the polysaccharide to a monosaccharide, an oligosaccharide, or both.

In one embodiment of the invention, the biomolecule of interest is an enzyme converting the lipid to a fatty acid, a monosaccharide, or both. In one embodiment of the invention, the monosaccharide or oligosaccharide is oligoalginate, mannuronate, guluronate, mannitol, a-keto acid, 4-deoxy-L-erythro-hexoselulose uronate (DEHU), 2-keto-3-deoxy D-gluconate (KDG), glucose, glucuronate, galacturonate, galactose, xylose, arabinose, or mannose. In one embodiment of the invention, the fatty acid is 14:0, trans-14, 16:0, 16:ln-7, trans-16, 16:2n-6, 18:0, 18:ln-9, 18:2n-6, 18:3n-6, 18:3n-3, 18:4n-3, 20:0, 20:2n-6, 20:3n-6, 20:4n-3,20:4n-6, or 20:5n-3.

In one embodiment of the invention, the biomolecule of interest is an enzyme converting the extracellular biomolecule to a commodity chemical. In one embodiment of the invention, the commodity chemical is ethanol, butanol, or biodiesel. In one embodiment of the invention, the biodiesel is a fatty acid, a fatty acid ester, or a terpenoid.

As used herein, the term "inactivated" refers to the interruption or to the suppression of the expression of a gene at transcriptional or translational levels. Preferably, the term "inactivated" refers to a gene whose transcription is suppressed.

According to the invention, the inactivation of a gene may be due to the mutation of the gene or to the insertion of the expression system within the coding sequence of the gene.

In the meaning of the present invention, the term "mutation" refers to a stable change in the genetic sequence. Examples of mutation which could lead to the inactivation of a gene in the present invention include, but are not limited to, point mutations, insertions, deletions and amplification or gene duplication.

Preferably, the mutation is a deletion. The term "deletion" as used herein means the loss or absence of a gene, preferably the total loss or absence of a gene. More preferably, the deletion starts at or before the start codon of the deleted gene, and ends at or after the stop codon of the deleted gene.

In one embodiment of the invention, the *S* gene is inactivated. The *S* gene encodes both a holing (S105) and an anti-holin (S107) protein. The holin protein triggers the formation of holes in the membrane. The holing is required for release of the endolysin encoded by the *R* gene. At the opposite, the antiholin protein inhibits the S105 hole formation. According to an embodiment, the S gene has the sequence SEQ ID NO: 1. In another embodiment, the sequence of the *S* gene presents a sequence identity of at least 70%, at least 75%, at least 80%, at least 85%, at least 90%, at least 91%, at least 92%, at least 93%, at least 94%, at least 95%, 5 at least 96%, at least 97%, at least 98%, and even more preferably of at least 99% with SEQ ID NO: 1.

The *S* gene may contain conservative sequence modifications that refer to amino acid modifications that do not significantly affect or alter the function of the *S* protein. Such conservative modifications include amino acid substitutions, additions and deletions. Modifications can be introduced into the *S* gene sequence by standard techniques known in the art, such as site -directed mutagenesis and PCR-mediated mutagenesis. Conservative amino acid substitutions are typically those in which an amino acid residue is replaced with an amino acid residue having a side chain with similar physicochemical properties. The modified sequence of the *S* protein may comprise one, two, three, four or more amino acid insertions, deletions or substitutions. Where substitutions are made, preferred substitutions will be conservative modifications. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g. glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g. threonine, valine, isoleucine) and aromatic side chains (e.g., tyrosine, phenylalanine, tryptophan, histidine). Thus, one or more amino acid residues within the sequence of the *S* protein can be replaced with other amino acid residues from the same side chain family and the modified *S* protein can be tested for retained function (i.e., the properties set forth herein) by comparison with the *S* protein encoded by the sequence SEQ ID NO: 1.

The term "identity" or "identical", when used in a relationship between the sequences of two or more polypeptides, refers to the degree of sequence relatedness between polypeptides, as determined by the number of matches between strings of two or more amino acid residues. "Identity" measures the percent of identical matches between the smaller of two or more sequences with gap alignments (if any) addressed by a particular mathematical model or computer program (i.e., "algorithms"). Identity of related polypeptides can be readily calculated by known methods. Such methods include, but are not limited to, those described in Computational Molecular Biology, Lesk, A. M., ed., Oxford University Press, New York, 1988; Biocomputing: Informatics 5 and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988). Preferred methods for determining identity are designed to give the largest match between the sequences tested. Methods of determining identity are described in publicly available computer programs. Preferred computer program methods for determining identity between two sequences include the GCG program package, including GAP (Devereux et al., Nucl. Acid. Res. \2, 387 (1984); Genetics Computer Group, University of Wisconsin, Madison, Wis.), BLASTP, BLASTN, and FASTA (Altschul et al., J. Mol. Biol. 215, 403-410 (1990)). The BLASTX program is publicly available from the National Center for Biotechnology Information (NCBI) and other sources (BLAST Manual, Altschul et al. NCB/NLM/NIH Bethesda, Md. 20894; Altschul et al., supra). The well-known Smith Waterman algorithm may also be used to determine identity.

In another embodiment, the *R* gene is inactivated. The R protein is an endolysin: this transglycosylase degrades the murein of the cell wall of the host cell. According to an embodiment, the *R* gene has the sequence SEQ ID NO: 2. In another embodiment, the sequence of the *R* gene presents a sequence identity of at least 70%, at least 75%, at least 80%, at least 85%, at least 90%, at least 91%, at least 92%, at least 93%, at least 94%, at least 95%, 5 at least 96%, at least 97%, at least 98%, and even more preferably of at least 99% with SEQ ID NO: 2.

The *R* gene may contain conservative sequence modifications as described here above that refer to amino acid modifications that do not significantly affect or alter the function of the *R* protein. The modified sequence of the *R* protein may comprise one, two, three, four or more amino acid insertions, deletions or substitutions. Thus, one or more amino acid residues within the sequence of the *R* protein can be replaced with other amino acid residues from the same side chain family and the modified *R* protein can be tested for retained function (i.e., the properties set forth herein) by comparison with the *R* protein encoded by the sequence SEQ ID NO: 2.

In another embodiment, the *Rz* gene is inactivated. The Rz protein belongs to the spanin family. This protein may be involved in disrupting the outer membrane of the host cell during the lytic phase. According to an embodiment, the *Rz* gene has the sequence SEQ ID NO: 3. In another embodiment, the sequence of the *Rz* gene presents a sequence identity of at least 70%, at least 75%, at least 80%, at least 85%, at least 90%, at least 91%, at least 92%, at least 93%, at least 94%, at least 95%, 5 at least 96%, at least 97%, at least 98%, and even more preferably of at least 99% with SEQ ID NO: 3.

The *Rz* gene may contain conservative sequence modifications as described here above that refer to amino acid modifications that do not significantly affect or alter the function of the *Rz* protein. The modified sequence of the *Rz* protein may comprise one, two, three, four or more amino acid insertions, deletions or substitutions. Thus, one or more amino acid residues within the sequence of the *Rz* protein can be replaced with other amino acid residues from the same side chain family and the modified *Rz* protein can be tested for retained function (i.e., the properties set forth herein) by comparison with the *Rz* protein encoded by the sequence SEQ ID NO: 3.

In another embodiment, a nucleic acid fragment comprising the coding sequence of the S gene is deleted. According to the invention, said nucleic acid fragment does not comprise the coding sequences of the *ral* gene (SEQ ID NO: 4) and/or of the *N* gene (SEQ ID NO: 5). Therefore, according to an embodiment, when the phage is lambda, the region between residues 33930 and 35582 is not deleted.

According to another embodiment, when the phage is lambda(DE3), the region between residues 10813 and 11391 is not deleted.

Preferably, said nucleic acid fragment does not comprise the coding sequence of *N* and *C2* (SEQ ID NO: 6), as well as the regulatory sequences of the promoter of *C2*. To make sure that the regulatory sequences of C2 are not deleted, the fragment to be deleted may begin at the start codon ATG of the following gene: the Cro gene.

More preferably, said nucleic acid fragment does not comprise the coding sequence of *ral, N* and *C2* (SEQ ID NO: 6), as well as the regulatory sequences of the promoter of *C2.*

The *C2* gene encodes a repressor protein, which is important for maintaining the lysogenic state. This gene is also called cI in the sequence of several other lambdoid phages. In the lambda phage, the coding sequence of the *ral, N* and *cI* genes is from residues 33930 to 38040. In the lambda DE3 phage, the coding sequence of the *ral, N, C2* genes is from residues 10813 to 12436.

In one embodiment, the length of said deleted nucleic acid fragment is from 30 kb to 300 b, preferably from 5 kb to 500 b. In another embodiment, the length of said deleted nucleic acid fragment is 30 kb, 25 kb, 20 kb, 15 kb, 10 kb, 9 kb, 8 kb, 7 kb, 6 kb, 5 kb, 4 kb, 3 kb, 2kb, 1 kb, 750 b, 500b.

According to one embodiment where the phage is lambda, the deleted nucleic acid fragment starts at a position ranging from the position 35582 to the position 45186, preferably from 38041 to 45186 and ends at a position ranging from the position 45510 to the position 48502

According to another embodiment where the phage is lambda DE3, the deleted nucleic acid fragment starts at a position ranging from the position 11392 to the position 16764, preferably from 12437 to the position 16764 and ends at a position ranging from 17088 and 42925.

In another embodiment, the nucleic acid fragment to be deleted comprises at least the coding sequence of the *S* and *R* genes. According to one embodiment, when the phage is lambda, the nucleic acid fragment starts at a position ranging from the position 35582 to the position 45186, preferably from 38041 to 45186, and ends at a position ranging from the position 45970 to the position 48502. In another embodiment, when the phage is lambda DE3, the nucleic acid fragment starts at a position ranging from the position 11392 to the position 16764, preferably from 12437 to the position 16764, and ends at a position ranging from 17548 and 42925.

In another embodiment, the nucleic acid fragment to be deleted comprises at least the coding sequence of the *S, R* and *Rz* genes. According to one embodiment, when the phage is lambda, the nucleic acid fragment starts at a position ranging from the position 35582 to the position 45186, preferably from 38041 to 45186. According to this embodiment, the fragment may end at a position ranging from the position 46428 to the position 46458. Still according to this embodiment, the nucleic acid fragment may end at a position ranging from 46128 to 48502.

According to another embodiment, when the phage is lambda DE3, the nucleic acid fragment starts at a position ranging from the position 11392 to the position 16764, preferably from 12437 to the position 16764. According to this embodiment, the fragment may end at a position ranging from position 18006 and 18036. Still according to this embodiment, the nucleic acid fragment may end at a position ranging from position 18006 to 24496. Still according to this embodiment, the nucleic acid fragment may end at a position ranging from position 18006 to 42925.

According to another embodiment, the nucleic acid fragment to be deleted comprises at least the coding sequence of the *R* gene.

According to one embodiment, when the phage is lambda, the nucleic acid fragment starts at a position ranging from the position 35582 to the position 45493, preferably ranging from the position 38041 to the position 45493. According to this embodiment, the nucleic acid fragment to be deleted may end at a position ranging from 45970 to the position 48502.

According to another embodiment, when the phage is lambda DE3, the nucleic acid fragment starts at a position ranging from the position 11392 to the position 17071, preferably ranging from the position 12437 to the position 17071. According to this embodiment, the nucleic acid fragment to be deleted may end at a position ranging from 17548 to the position 24496. Still according to this embodiment, the nucleic acid fragment to be deleted may end at a position ranging from 17548 to the position 42925.

According to another embodiment, the nucleic acid fragment to be deleted comprises at least the coding sequence of the *R* and *Rz* genes.

According to one embodiment, when the phage is lambda, the nucleic acid fragment starts at a position ranging from the position 35582 to the position 45493, preferably ranging from the position 38041 to the position 45493. According to this embodiment, the nucleic acid fragment to be deleted may end at a position ranging from 46428 to the position 46458. Still according to this embodiment, the nucleic acid fragment to be deleted may end at a position ranging from 46428 to the position 48502.

According to another embodiment, when the phage is lambda DE3, the nucleic acid fragment starts at a position ranging from the position 11392 to the position 17071, preferably ranging from the position 12437 to the position 17071. According to this embodiment, the nucleic acid fragment to be deleted may end at a position ranging from 18006 to the position 18330. Still according to this embodiment, the nucleic acid fragment to be deleted may end at a position ranging from 18006 to the position 24496. Still according to this embodiment, the nucleic acid fragment to be deleted may end at a position ranging from 18006 to the position 42925.

In one embodiment, the *Int* gene is inactivated. The Int protein manages the insertion and the excision of phage genome into the host's genome. According to an embodiment, the *Int* gene has the sequence SEQ ID NO: 7. In another embodiment, the sequence of the *Int* gene presents a sequence identity of at least 70%, at least 75%, at least 80%, at least 85%, at least 90%, at least 91%, at least 92%, at least 93%, at least 94%, at least 95%, 5 at least 96%, at least 97%, at least 98%, and even more preferably of at least 99% with SEQ ID NO: 7.

The *Int* gene may contain conservative sequence modifications as described here above that refer to amino acid modifications that do not significantly affect or alter the function of the Int protein. The modified sequence of the Int protein may comprise one, two, three, four or more amino acid insertions, deletions or substitutions. Thus, one or more amino acid residues within the sequence of the Int protein can be replaced with other amino acid residues from the same side chain family and the modified Int protein can be tested for retained function (i.e., the properties set forth herein) by comparison with the Int protein encoded by the sequence SEQ ID NO: 7.

In one embodiment, the *Xis* gene is inactivated. The Xis protein is an excisionase, which is involved in the process of excision of the lambda phage DNA out of the bacterial host chromosome. According to an embodiment, the *Xis* gene has the sequence SEQ ID NO: 8. In another embodiment, the sequence of the *Xis* gene presents a sequence identity of at least 70%, at least 75%, at least 80%, at least 85%, at least 90%, at least 91%, at least 92%, at least 93%, at least 94%, at least 95%, 5 at least 96%, at least 97%, at least 98%, and even more preferably of at least 99% with SEQ ID NO: 8.

The *Xis* gene may contain conservative sequence modifications as described here above that refer to amino acid modifications that do not significantly affect or alter the function of the Xis protein. The modified sequence of the Xis protein may comprise one, two, three, four or more amino acid insertions, deletions or substitutions. Thus, one or more amino acid residues within the sequence of the Xis protein can be replaced with other amino acid residues from the same side chain family and the modified Xis protein can be tested for retained function (i.e., the properties set forth herein) by comparison with the Xis protein encoded by the sequence SEQ ID NO: 8.

In one embodiment, a nucleic acid fragment comprising the coding sequence of the *Int* gene is deleted. According to an embodiment, the phage is lambda and the nucleic acid fragment to be deleted comprises the residues from position 27812 to 28882. According to another embodiment, the phage is lambda and the nucleic acid fragment to be deleted starts at a position ranging from position 1 to position 27812; and ends at a position ranging from 28882 to 33929.

In one embodiment, a nucleic acid fragment comprising the coding sequence of the *Xis* gene is deleted.

According to one embodiment, the phage is lambda and the deleted nucleic acid fragment to be deleted comprises the residues from position 28860 to 29078. According to another embodiment, the phage is lambda and the fragment starts at a position ranging from position 1 to position 28860, and ends at a position ranging from position 29078 to position 33929.

According to another embodiment, the phage is DE3 and the deleted nucleic acid comprises the residues from position 5586 to 5804. According to another embodiment, the fragment to be deleted starts at a position ranging from position 1 to position 5586, and ends at a position ranging from position 5804 to position 10812.

In one embodiment, a nucleic acid fragment comprising the coding sequences of the *Xis* and *Int* genes is deleted. According to an embodiment, the phage is lambda and the nucleic acid fragment to be deleted comprises the residues from position 27812 to 29078. According to another embodiment, the phage is lambda and the nucleic acid fragment to be deleted starts at a position ranging from position 1 to position 27812; and ends at a position ranging from 29078 to 33929.

According to an embodiment, the *attP* sequence is not deleted from the sequence of the phage of the invention. The *attP* sequence is SEQ ID NO: 9. The attP sequence is located from position 27586 and 27817 in the sequence of the Lambda Phage, and from position 42788 to 42925 and from 1 to 94 in the genome of the Lambda DE3 phage (the genome of the phage is circular, the attP sequence is thus continue).

In one embodiment of the invention, the genetically modified phage comprises the *attP* sequence and the sequence of the *C2* gene. In another embodiment, the genetically modified phage consists of the *attP* sequence and the sequence of the *C2* gene.

According to a preferred embodiment, the genetically modified phage of the invention has the sequence SEQ ID NO: 10.

The present invention also relates to a process for producing the modified phage of the invention, wherein said process comprises at least two steps of deletion of genes.

In an embodiment, the process of the invention is carried out with a phage integrated in the genome of a host cell.

In an embodiment, the host cell is a microorganism, preferably a prokaryote, more preferably a bacterium, more preferably a gram negative bacterium. Advantageously, the host cell is a bacterium from the *Enterobacteriacea* family according to the current applicable taxonomy. If the taxonomy should change, the skilled artisan would now how to adapt the changes in the taxonomy to deduce the strains that could be used in the present invention. Examples of bacteria from the *Enterobacteriacea* family include, but are not limited to, bacteria belonging to the genera *Escherichia, Enterobacter, Erwinia, Klebsiella, Pantoea, Photorhabdus, Providencia, Salmonella, Serratia, Shigella, Morganella* and *Yersinia.* According to a preferred embodiment, the host cell belongs to the *Escherichia* genus, and more preferably the host cell is *Escherichia coli* (*E. coli*).

Methods for deleting genes from an integrated phage are well known to the skilled artisan. Examples of such methods include, but are not limited to homologous recombination (also called recombineering) using the lambda Red-encoded genes: exo, bet and gam. It is also possible to use the corresponding recE and recT genes from the prophage Rac. The genes exo and recE encode a 5'-3' exonuclease that produces 3' overhangs. The bet and recT genes encode a pairing or also called annealing protein that binds the 3' overhangs and mediates its annealing and homologous recombination between two complementary DNA sequences. The gam gene encodes an inhibitor of the E. coli RecBCD exonuclease and thus protects linear DNA fragments of interest. The method of recombineering was well described by several researchers including Datsenko and Wanner (PNAS 97-12, 6640-6645, 2000) and Stewart et al (WO0104288). The principle of the method is to generate (by PCR amplification for example) a DNA fragment containing the fragment to integrate and two recombination arms. These arms are homologous to the regions adjacent to the gene to be inactivated. They will be used to target the insertion of the fragment of interest. It is possible to create this kind of DNA fragment by PCR using primers containing homologous arms from 20 to 60 nucleotides.

The present invention also relates to a host cell comprising the genetically modified phage of the invention. In a preferred embodiment, the host cell of the invention comprises the genetically modified phage integrated in its genome.

In one embodiment, the host cell is a microorganism, preferably a prokaryote, more preferably a bacterium, more preferably a gram negative bacterium. Advantageously, the host cell is a bacterium from the *Enterobacteriacea* family according to the current applicable taxonomy. If the taxonomy should change, the skilled artisan would now how to adapt the changes in the taxonomy to deduce the strains that could be used in the present invention. Examples of bacteria from the *Enterobacteriacea* family include, but are not limited to, bacteria belonging to the genera *Escherichia, Enterobacter, Erwinia, Klebsiella, Pantoea, Photorhabdus, Providencia, Salmonella, Serratia, Shigella, Morganella* and *Yersinia.* According to a preferred embodiment, the host cell belongs to the *Escherichia* genus, and more preferably the host cell is *Escherichia coli* (*E. coli*). Examples of strains of *E. coli* which could be used in the present invention include, but are not limited to, strains derived from E. Coli K-12 and E. coli B, such as, for example, MG1655, Top10, DH10B, DH5alpha, HMS174, BL21, BL21(DE3), BL21(DE3) pLysS, BL21(DE3) pLysE.

In one embodiment, genes of the host cells may be inactivated.

In one embodiment, the gene *tonA* (also known as *fhuA,* SEQ ID NO: 11) is inactivated. The TonA/FhuA protein is a receptor for the phages T1, T5 and Phi80.

In one embodiment, the gene *galK* (SEQ NO: 12) is inactivated. The deletion of this gene allows the use of the *galK* positive/negative selection for deletion of genes by a method based on homologous recombination.

In one embodiment, the gene *araB* (SEQ NO: 13) is inactivated. In another embodiment, the gene *araA* (SEQ ID NO: 14) is inactivated. The inactivation of *araB* and/or *araA* is required for the use of the promoter PBAD (inducible by arabinose) within the host cell.

In one embodiment, the gene *lon* (SEQ ID NO: 15) and/or the gene *ompT* (SEQ ID NO: 16) are inactivated. The Lon protein is an ATP dependent protease. The OmpT protein is an outer membrane protease. Preferably, the genes *lon* and *ompT* are inactivated.

In one embodiment, the gene *rcsA* (SEQ ID NO: 17) is inactivated. The protein RcsA is a positive regulator of the synthesis of the capsule, which is degraded by the Lon protease.

In one embodiment, the gene *hsdR* (SEQ ID NO: 18) and/or the gene *mrr* (SEQ ID NO: 19) are inactivated. The HsdR and Mrr proteins are restriction enzymes with different specificity. Preferably, the genes *hsdR* and *mrr* are both inactivated.

In one embodiment, the gene *endA* (SEQ ID NO: 20) and/or the gene *recA* (SEQ ID NO: 21) are inactivated. EndA is a DNA specific endonuclease. RecA is a recombination protein with protease and nuclease activity. Preferably, the genes *endA* and *recA* are both inactivated.

In one embodiment of the invention, at least one of the genes *tonA, galK, araB, araA, lon, ompT, rcsA, hsdR, mrr, endA* and *recA* are inactivated. Preferably, the inactivated genes are deleted.

In a preferred embodiment, the genes tonA, galK, araB, lon, ompT, rcsA, hsdR, mrr, endA and recA are inactivated. Preferably, the genes tonA, galK, araB, lon, ompT, rcsA, hsdR, mrr, endA and recA are deleted.

In one embodiment of the invention, the host cell of the invention is transformed with a nucleic acid sequence encoding a toxic molecule, as described in US2004/0115811. The presence of the nucleic acid sequence encoding the toxic molecule will allow the selection of recombinant clones having integrated a gene of interest and a nucleotide sequence encoding a functional antidote protein to a toxic molecule, wherein said recombinant clones are the ones which survive following their integration into a host cell comprising in its genome a nucleotide sequence encoding said toxic molecule.

According to a preferred embodiment of the present invention, the antidote protein and the toxic molecule are respectively, an anti-poison protein and a poison protein. Said anti-poison or poison proteins could be wild type proteins or modified proteins which are naturally or artificially poisonous and affect one or more vital functions of a cell (preferably, a prokaryote cell) and may lead to the killing of the cell.

The antidote protein and the toxic molecule are preferably selected from the group consisting of CcdA/CcdB proteins, Kis/Kid proteins, Phd/Doc proteins, SoK/HoK proteins, RelB/relE proteins, PasB (or PasC)/PasA proteins, mazF/mazE proteins as described in US2004/0115811, or any other couple of anti-poison/poison molecules which are or are not of plasmid origin. The toxic molecule can also be a toxin protein being naturally or artificially toxic and affecting one or more vital functions of a (prokaryote) cell. The protein encoded by the gene sacB (from Bacillus amylolique-faciens), the protein GpE, the protein GATA-1 and the protein Crp are other examples of such toxic molecules. The gene *sacB* encodes the levan sucrase which catalyses the hydrolysis of sucrose into products which are toxic for E. Coli (Pierce et al. Proc. Natl. Acad. Sci., Vol. 89, N[deg.]6 (1992) p. 2056-2060). The protein GpE encodes the E genes from the bacteriophage [phi]X174 which includes six unique restriction sites and encodes gpE and which causes lysis of E. Coli cell (Heinrich et al., Gene, Vol. 42 n[deg.]3 (1986) p. 345-349). The protein GATA-1 has been described by Trudel et al. (Biotechniques 1996, Vol. 20(4), p. 684-693). The protein Crp has been described by Schlieper et al. (Anal. Biochem. 1998, Vol. 257(2), p. 203-209).

The antidote proteins to said toxic molecule are any protein able to reduce or suppress the effect of the corresponding toxic molecule on a cell (preferably a prokaryotic cell), when said toxic molecule is produced by said cell.

According to a preferred embodiment, the host cell of the invention comprises a nucleic acid sequence encoding the protein CcdB. The *ccdB* gene has the sequence SEQ ID NO: 22.

The present invention also relates to a kit comprising a host cell as hereinabove described, wherein the gene encoding a poison protein is inserted, and a plasmid carrying the nucleic acid sequence of the gene encoding the anti-poison protein. The expression of the anti-poison protein in the host cell is required for maintaining the viability of the host cell. In a preferred embodiment, the poison protein is encoded by the *ccdB* gene, and the anti-poison protein is encoded by the *ccdA* gene (SEQ ID NO: 23).

According to an embodiment of the invention, the plasmid of the kit may further contain, or may be modified to further contain, the nucleic acid sequence of the biomolecule of interest.

The present invention also refers to a process for preparing a host cell as hereinabove described.

In one embodiment, the process of the invention comprises a step of infection of the host cell by a genetically modified phage according to the invention. In one embodiment of the invention, said infection step includes the use of a helper phage. In the meaning of the present invention, the term "helper phage" refers to a phage used to complement a deletion or an inactivation of another phage. The helper phage will provide the missing functions to another phage to be able to infect bacteria or to prepare phage stock. Usually, the helper phage cannot form a lysogen by itself because it is cI minus (it has no repressor and is thus virulent).

The process for infecting a host cell with a phage using a helper phage is well known in the art. The first step is the preparation of the lysates and the second one is the lysogenization. Briefly, the bacterial lysates of the helper phage are prepared using standard methods as described in "Molecular cloning: a laboratory manual", Sambrook et al (2001, ISBN 978-087969577-4) or in "Large- and Small-Scale Preparation of Bacteriophage lambda lysate and DNA", Su et al, BioTechniques 25:44-46 (July 1998). Preparation of the phage of interest is done using the same principle, after phage induction (most often using UV irradiation or any situation where a lysogen undergoes DNA damage or the SOS response of the host or Cro production) in order to launch the lytic cycle and using a helper phage to provide the missing functions. An alternative to the helper phage is the use of a plasmid encoding the missing functions.

Next, the phage lysates are mixed with the targeted bacteria and plated on LB plates in order to get lysogens (as described in lambda DE3 lysogenization kit from Novagen, User Protocol TB031 or an alternative method is described in Studier and Moffat, Journal of Molecular Biology, 1986, 189:113-130). A selection phage can be used to select specifically bacteria containing the phage of interest. This selection phage is a virulent phage having the same immunity as the phage of interest. Consequently, the selection phage is unable to form plaques or to kill bacteria lysogens for the phage of interest because this phage produces the cI repressor (also called C2 in DE3 lambda phage).

The present invention thus also relates to a kit comprising the modified phage of the invention, as hereinabove described, and a helper phage. Examples of helper phages include, but are not limited to, helper phage B10 or any other lambdoid phage with a different immunity than the phage of interest (example: phage with immunity 434, 80,...). Some helper phages and their manipulations were well described in the literature including in Haldimann and Wanner (Journal of Bacteriology, 183, 6384-6393,2001).

In one embodiment, the process for preparing a host cell of the invention further comprise a step of deletion, wherein nucleic acid sequences of the host cell are deleted. Methods for deleting the sequence of a gene are well known by the skilled artisan. The more efficient method isthe homologous recombination method mediated by the lambda Red-encoded genes or the recE and recT genes from the prophage Rac.. As described above, this method was well described by several researchers including Datsenko and Wanner (PNAS 97-12, 6640-6645, 2000) and Stewart et al (WO0104288 PCR products are generated using primers with 20- to 60-nt extensions that are homologous to regions adjacent to the gene to be inactivated. Since only a small amount of bacteria will effectively recombine the fragment of interest, it is necessary to have a strong selection marker to select it. Antibiotic markers can be used to select the recombinants: the modified primers are used to amplify an antibiotic resistance gene. After transformation and activation of the recombination genes, recombinant bacteria are selected on medium containing the appropriate antibiotic. In this case, the targeted gene is replaced by an antibiotic resistance gene. In order to use the same strategy for the next deletion, it is necessary to remove this antibiotic resistance gene during a second step. As described in Datsenko and Wanner, it is possible to use antibiotic resistance gene that are flanked by FRT (FLP recognition target) sites. The resistance genes are then eliminated by using a helper plasmid encoding the FLP recombinase. The antibiotic resistance gene is removed by this site-specific recombinase but this method leaves traces: one site-specific recombination site is still present after removal of the antibiotic resistance gene.

To avoid the presence of this site, more preferably, the method uses *galK* as a marker gene. The principle of the galK selection is described in Warming et al. (Nucleic acid research, 2005, 33(4)). This method uses galK as a positive selection marker (growth on minmal medium containing galactose) during the first recombination (insertion). The removal of this marker is performed during a second homologous recombination step. During this step, galK is used as a negative selection marker on minimal medium containing 2-deoxy-galactose (DOG). The galK gene product, galactokinase, catalyzes the first step in the galactose degradation pathway. Galactokinase also efficiently catalyzes the phosphorylation of the DOG galactose analog. The product of this reaction cannot be further metabolized, leading to the accumulation of a toxic molecule (2-deoxy-galactose-1-phosphate). The advantage of this method is to avoid the presence of specific recombination site after deletion of the targeted gene and removal of the selective marker.

The present invention also relates to a process for producing a biomolecule of interest, comprising:
- cultivating a host cell comprising the genetically modified phage according to the invention and the nucleic acid sequence of the biomolecule of interest,
- recovering the biomolecule of interest.

In one embodiment of the invention, the nucleic acid sequence of the biomolecule of interest is comprised within the expression system of the genetically modified phage. According to this embodiment, the production of the biomolecule of interest is direct, i.e. results from the expression of the gene of the expression system, for example by culture in a medium wherein the promoter comprised in the expression system is induced.

In another embodiment of the invention, the expression system of the genetically modified phage comprises the nucleic acid sequence of the T7 RNA polymerase under the control of a lac promoter, preferably the lacUV5 promoter. According to this embodiment, the process for producing the biomolecule of interest comprises the transformation of the host cell with a plasmid comprising the nucleic acid sequence of the biomolecule of interest under the control of a the T7 promoter. The expression from the T7 promoter is under the control of T7 RNA polymerase, which is stringently specific for the T7 promoter, i.e. the T7 promoter can only be utilized by the RNA polymerase of bacteriophage T7. When IPTG is added to the culture medium, T7 RNA polymerase is expressed by transcription from the lac promoter, and allows the expression of the biomolecule of interest.

In the meaning of the present invention, the term "T7 promoter" includes promoters that are present in the genome of bacteriophage T7, as well as consensus sequences and variants of such promoters with the ability to mediate transcription by the T7 RNA polymerase. The bacteriophage T7 contains seventeen different promoter sequences, all of which comprise a highly conserved nucleotide sequence.

According to a preferred embodiment, the plasmid comprising the nucleic acid sequence of the biomolecule of interest also comprises the nucleic acid sequence of *ccdA,* and the host cell comprises the sequence of *ccdB* integrated in its genome. Therefore, only recombinant clones containing the plasmid are propagated.

According to one embodiment, the biomolecule of interest is secreted by the host cell in the fermentation broth. According to this embodiment, the biomolecule of interest may be easily recovered from the fermentation broth.

According to another embodiment, the biomolecule of interest is not secreted by the host cell in the fermentation broth. Methods for recovering an intracellular biomolecule of interest are well-known in the art. Examples of such method include, but are not limited to, the use of trichloroacetic acid (TCA) or cracking buffer containing sodium dodecyl sulfate (SDS) to recover total cytoplasmic proteins in denaturing conditions or the use of sonication, French press or equivalent to disrupt bacteria under pressure in order to recover total cytoplasmic proteins in native (not denaturing) conditions. Next, the protein of interest can be purified using specific methods including but not limited to the use of affinity or ion exchange columns.

### BRIEF DESCRIPTION OF THE DRAWINGS

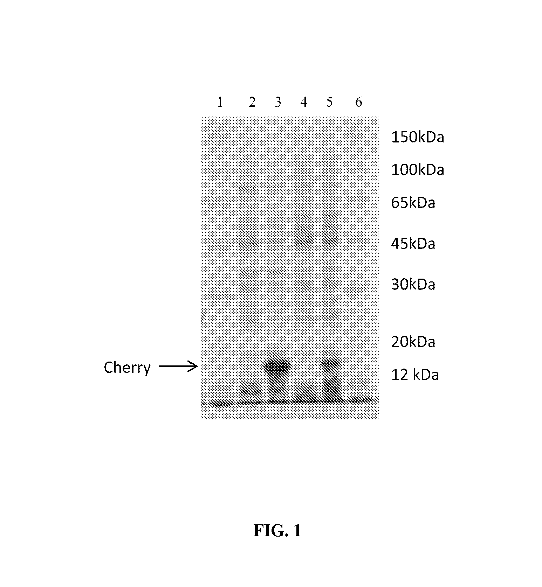

**Figure 1****: Picture of a SDS-Page gel colored with Coomassie blue staining, showing the production of the protein of interest.** 1 and 6: size; 2: pellet [pScherryl] M11DE3 before induction; 3: pellet [pScherryl] M11DE3 after induction; 4: pellet [pscherryl] HMS174DE3 before induction; 5: pellet [pscherryl] HMS174DE3 after induction. The array is for identifying the protein of interest.

### EXAMPLES

The present invention is further illustrated by the following examples.

### Example 1: Deletion of the xis, exo, bet, gam, kil, cIII, N and ral genes

### a) Deletion of xis DNA region and replacement by galK

First the galK gene was amplified by polymerase chain reaction (PCR) on pGalK plasmid using the primers XisgalKstart (5' *GGGGGTAAATCCCGGCGCTCATGACTTCGCCTTCTTCCCA*GAATTCCTGTTGAC AATTA 3', SEQ ID NO: 24) and XisgalK stop (5' *GTTCTGATTATTGGAAATCTTCTTTGCCCTCCAGTGTGAG*CAGCACTGTCCTGCT CCTTG 3', SEQ ID NO: 25). These primers contain at the 5' end a sequence of 40 bases identical to the DNA target (italicized). These sequences of 40 bases are the recombination arms. The 3' ends were designed to amplify the *galK* gene and its constitutive promoter. The DNA fragment amplified by PCR (1315bp) targeted the genes *xis, exo, bet, gam, kil* and *cIII* (DNA fragment of 5133bp): these genes were replaced during homologous recombination by the galK gene and its promoter. Electrocompetent bacteria carrying the T7(DE3) prophage and the pKD46 plasmid were prepared according to Datsenko and Wanner (PNAS 97-12, 6640-6645, 2000). Next, the amplified galK fragment was electroporated in these bacteria according to standard procedures (200ng of DNA fragment was used for each electroporation). SOC medium was added and bacteria were incubated during 1 hour at 37°C. Next, bacteria were washed (centrifuged, medium removal, addition of fresh medium and resuspension) twice with M9 minimal medium (Sambrook et al (2001, ISBN 978-087969577-4)) and plated on bacterial plates containing minimal M9 medium and 1% galactose. Plates were incubated at 37°C during 1 or 2 days.

The next step was the bacterial screening: PCR screening was performed directly on colonies using the Xis1 (5'GTCTTCAAGTGGAGCATCAG3', SEQ ID NO: 26) and Xis4 (5'ACCAGGACTATCCGTATGAC3', SEQ ID NO: 27) primers. An amplification of a 5774bp DNA fragment corresponds to a non-modified chromosome (non-recombinant colony) and, on the contrary, an amplification of a 1955bp DNA fragment corresponds to a recombinant chromosome. Bacteria allowing amplification of the 1955bp DNA fragment were selected and streaked two times on selective plates (minimal M9 medium supplemented with 1% galactose) in order to purify it and to remove possible unmodified copies of the chromosome. The PCR screening was done one more time at the end of the purification step and 3 bacteria allowing amplification of the 1955bp DNA fragment were selected. The amplified DNA fragments corresponding to these bacteria were sequenced using the same primers in order to confirm the DNA recombination and the deletion of the Xis DNA region (*xis, exo, bet, gam, kil* and *cIII* genes).

### b) GalK removal

A DNA fragment containing large recombination arms (of 350bp and 343 bp) was constructed by PCR to remove galK and the N and ral genes. The first arm was amplified byPCR on bacterial colonies containing the T7(DE3) prophage using the Xis1 and Xis2 (5'CCAAACGGAACAGATGAAGAAGGCGAAGTCATGAG3', SEQ ID NO: 28) primers. A DNA fragment of 365 bases was amplified. The second arm was also performed by PCR on the same bacteria using the Xis3 (5' GACTTCGCCTTCTTCATCTGTTCCGTTTGGCTTCC3', SEQ ID NO: 29) and Xis6 (5' GTAATGGAAAGCTGGTAGTCG3', SEQ ID NO: 30) primers. A DNA of fragment of 358 bases was amplified. Both recombination arms were purified after agarose gel electrophoresis. Xis2 and Xis3 primers were designed to generate DNA fragments containing an identical sequence of 30 base pairs. This sequence was used to join both recombination arms in a third PCR using Xisl and Xis6 primers. A DNA fragment of 693 bp was generated. This DNA fragment was electroporated in bacteria selected above and carrying the pKD46 plasmid (prepared as described in Datsenko and Wanner). SOC medium was added and bacteria were incubated at 37°C during 1 hour. Next, bacteria were washed twice with M9 medium and plated on selective plates containing M9 medium supplemented with 0,2% glycerol and 1% DOG. Plates were incubated during 2 days at 37°C. Several colonies were screened by PCR using the Xis5 (5' CAGCCGTAAGTCTTGATCTC3', SEQ ID NO: 31) and Xis7 (5'CAGCAGGCATGATCCAAGAG3', SEQ ID NO: 32) primers. An amplification of 3246bp corresponding to the unmodified DNA chromosome was always obtained instead of an amplification of 1122bp corresponding to the modified chromosome. The experiment was reproduced completely and independently three times without success: no bacteria comprising the desired deletion was obtained.

Consequently, we decided to remove only the GalK fragment and to leave the N and ral genes. The DNA fragment containing the recombination arms was generated as described above using Xis,1 Xis2b (5' TTTGCCCTCCAGTGTGAAGAAGGCGAAGTCATGAG3', SEQ ID NO: 33) for the first recombination arm (365bp) and Xis8 (5' CTCATGACTTCGCCTTCTTCACACTGGAGGGCAAAGAAG, SEQ ID NO: 34) and Xis4 for the second recombination arm (384bp). The joining PCR was performed using Xis1 and Xis4 primers and generated a DNA fragment of 714bp. This fragment was electroporated as described above in the bacteria selected and carrying the pKD46 plasmid. Bacteria were plated on the same selective plates containing DOG and incubated during two days at 37°C. PCR screening was performed using Xis5 and Xis6 primers. Bacteria showing an amplification of 1770bp (instead of an amplification of 3010bp corresponding to the unmodified chromosome) were selected and purified. The DNA fragment was sequenced using the Xis5 and Xis6 primers and showed the right removal of the galK gene. This recombination was performed only once since bacteria were obtained immediately.

These results show that it was not possible to remove galK associated to the N and ral genes using homologous recombination. However, using exactly the same procedure, we were able to remove galK alone.

In conclusion, we demonstrated that removal of the N and ral genes leads to the death of bacteria, which was unexpected.

### Example 2: Protein expression

In order to test the protein production efficiency of the strain constructed according to the invention, small-scale expression test were performed using bacteria called M11(DE3). The genotype of this strain is MG1655 ΔgalK, ΔrcsA, Δlon, ΔhsdR-mrr, ΔfhuA, ΔendA, ΔrecA, ΔaraB, ΔompT, λ-DE3 (T7pol, Δxis-ea10, ΔS-C). Since MG1655 is an Escherichia coli K-12, it was compared to another K-12 strain used as a standard in the field of protein production and called HSM174(DE3) (genotype: recA1, hsdR, λ-DE3, (Rif R)). Both strains were transformed with pSCherry1 plasmid DNA (Delphi Genetics, Belgium). This plasmid encodes a protein called "cherry" easily detectable (by eyes) under the control of the T7 promoter.

Protocol for a small-scale expression using IPTG:
1) Two Erlenmeyer flasks containing 10ml of LB medium were inoculated each with a single colony of the HMS174 (DE3) and the M11(DE3) carrying both the pSCherryl plasmid and incubated at 30°C overnight.
2) Two new flasks containing fresh medium were inoculated with 1ml of the overnight cultures and incubated with shaking at 37°C until OD600 reached 0.6.
3) A sample (1ml) from each flask was taken and centrifuged. The medium was discarded and the pellet was kept on ice. The samples were the non-induced controls. To induce protein expression in the remaining culture, IPTG (Isopropyl β-D-1-thiogalactopyranoside , 90µl of a fresh 100mM stock solution) was added to reach a final concentration of 1mM in both flasks. Incubation of both flasks was continued for 4 hours.
4) At the end of the induction period, the Optical Density at 600nm was measured for each culture (1.09 for M11(DE3) and 1.13 for HMS174(DE3)). A 1ml sample of each flask was centrigufed at maximum speed (13000 g) for 10 min at 4°C. It was observed that the pellet was red according to the expression of the Cherry protein. The supernatant was discarded and 100µl of water was added to resuspend the bacteria. 100µl of "cracking" buffer (100mM DTT, 2% SDS, 80mM Tris-HCl, pH 6.8, 0.006% bromophenol blue, 15% glycerol) was also added to lyse the bacteria. The non-induced samples were treated with the same protocol except that only 60µl of water and 60µl of cracking buffer were used (according to the optical density of the samples).
5) The samples were heated at 70°C-100°C (10min.) to resuspend all proteins and to denature the proteins.
6) 10 µl of each sample was loaded on 12% SDS-PAGE gel and migrated during 2 hours at 100 Volt.
7) After migration, the proteins were colored with Coomassie-blue staining.

As shown on figure 1, both starins were able to produce the protein of interest (Cherry protein, indicated by the array) but the production is about 5 to 10 times higher using M11(DE3) than using HMS174(DE3). The experiment was performed twice with exactly the same results.

## Claims

1. A genetically modified phage wherein
- an expression system is inserted,
- the *S* and/or the *R* genes are inactivated.

2. The genetically modified phage according to claim **1,** wherein the *Int* and/or *Xis* gene is inactivated.

3. The genetically modified phage according to claim **1** or **2,** wherein the *Rz* gene is inactivated.

4. The genetically modified phage according to any one of claims **1** to **3,** wherein
- an expression system is inserted,
- the *Int* gene, the *Xis* gene and the *R*, *S* and *Rz* genes are inactivated.

5. The genetically modified phage according to any one of claims **1** to **4,** wherein the expression system is the T7 expression system.

6. The genetically modified phage according to any one of claims **1** to **5,** wherein the phage is the lambda phage, the 434phage, the phi80 phage, the phi81 phage, the HK97 phage, the P21 phage.

7. The genetically modified phage according to any one of claim **1** to **6,** having the sequence SEQ ID NO: 10.

8. A kit comprising the genetically modified phage according to any one of claim **1** to **7** and a helper phage.

9. A host cell comprising the genetically modified phage according to any one of claims **1** to **7.**

10. The host cell according to claim **9,** wherein the host cell is an enterobacteria, preferably E. Coli.

11. The host cell according to any one of claim **9** to **10,** further comprising the inactivation of at least one of the genes *tonA, galK, araB, araA, lon, ompT, rcsA, hsdR, mrr, endA* and *recA.*

12. The host cell according to any one of claim **9** to **11,** comprising the insertion of the *ccdb* gene.

13. A kit comprising the host cell according to claim **12** and a plasmid comprising the *ccdA* gene.

14. A process for preparing the host cell according to any one of claim **9** to **12,** comprising infecting a host cell with a genetically modified phage according to any one of claims **1** to **7.**

15. A process for producing a biomolecule of interest, comprising
- cultivating a host cell comprising the genetically modified phage according to any one of claims **1** to **7** and the nucleic acid sequence of the biomolecule of interest,
- recovering the biomolecule of interest.


SEQUENCE LISTING

<110> DELPHI GENETICS SA
      Szpirer, Cédric

<120> GENETICALLY MODIFIED PHAGE AND USE THEREOF

<130> CV-123/EP

<160> 34

<170> BiSSAP 1.0

<210> 1
<211> 324
<212> DNA
<213> Enterobacteria phage lambda

<220>
<221> source
<222> 1..324
<223> /mol_type="DNA"
      /organism="Enterobacteria phage lambda"

<400> 1
atgaagatgc cagaaaaaca tgacctgttg gccgccattc tcgcggcaaa ggaacaaggc      60

atcggggcaa tccttgcgtt tgcaatggcg taccttcgcg gcagatataa tggcggtgcg     120

tttacaaaaa cagtaatcga cgcaacgatg tgcgccatta tcgcctggtt cattcgtgac     180

cttctcgact tcgccggact aagtagcaat ctcgcttata taacgagcgt gtttatcggc     240

tacatcggta ctgactcgat tggttcgctt atcaaacgct tcgctgctaa aaaagccgga     300

gtagaagatg gtagaaatca ataa                                            324


<210> 2
<211> 477
<212> DNA
<213> Enterobacteria phage lambda

<220>
<221> source
<222> 1..477
<223> /mol_type="DNA"
      /organism="Enterobacteria phage lambda"

<400> 2
atggtagaaa tcaataatca acgtaaggcg ttcctcgata tgctggcgtg gtcggaggga      60

actgataacg gacgtcagaa aaccagaaat catggttatg acgtcattgt aggcggagag     120

ctattcactg attactccga tcaccctcgc aaacttgtca cgctaaaccc aaaactcaaa     180

tcaacaggcg ccggacgcta ccagcttctt tcccgttggt gggatgccta ccgcaagcag     240

cttggcctga aagacttctc tccgaaaagt caggacgctg tggcattgca gcagattaag     300

gagcgtggcg ctttacctat gattgatcgt ggtgatatcc gtcaggcaat cgaccgttgc     360

agcaatatct gggcttcact gccgggcgct ggttatggtc agttcgagca taaggctgac     420

agcctgattg caaaattcaa agaagcgggc ggaacggtca gagagattga tgtatga        477


<210> 3

<211> 462
<212> DNA
<213> Enterobacteria phage lambda

<220>
<221> source
<222> 1..462
<223> /mol_type="DNA"
      /organism="Enterobacteria phage lambda"

<400> 3
atgagcagag tcaccgcgat tatctccgct ctggttatct gcatcatcgt ctgcctgtca      60

tgggctgtta atcattaccg tgataacgcc attacctaca aagcccagcg cgacaaaaat     120

gccagagaac tgaagctggc gaacgcggca attactgaca tgcagatgcg tcagcgtgat     180

gttgctgcgc tcgatgcaaa atacacgaag gagttagctg atgctaaagc tgaaaatgat     240

gctctgcgtg atgatgttgc cgctggtcgt cgtcggttgc acatcaaagc agtctgtcag     300

tcagtgcgtg aagccaccac cgcctccggc gtggataatg cagcctcccc ccgactggca     360

gacaccgctg aacgggatta tttcaccctc agagagaggc tgatcactat gcaaaaacaa     420

ctggaaggaa cccagaagta tattaatgag cagtgcagat ag                        462


<210> 4
<211> 201
<212> DNA
<213> Enterobacteria phage lambda

<220>
<221> source
<222> 1..201
<223> /mol_type="DNA"
      /organism="Enterobacteria phage lambda"

<400> 4
atgactacga ctattgataa aaatcaatgg tgtggacaat tcaagcgatg caatggatgc      60

aagctgcaat cggaatgcat ggttaagcct gaagaaatgt ttcctgtaat ggaagatggg     120

aaatatgtcg ataaatgggc aatacgaacg acggcaatga ttgccagaga acttggtaaa     180

cagaacaaca aagctgcctg a                                               201


<210> 5
<211> 300
<212> DNA
<213> Enterobacteria phage lambda

<220>
<221> source
<222> 1..300
<223> /mol_type="DNA"
      /organism="Enterobacteria phage lambda"

<400> 5
atggtaacca ttgtctggaa agaatccaaa ggtacggcaa aaagccgcta caaagctcgc      60

agagcagaac ttattgccga gcgacgcagt aatgaagcac tggcgcgaaa aattgcgcta     120

aagctctctg gttgcgtcag agcagataaa gcagcatcac tcggaagcct tcgctgcaag     180

aaggcagaag aagtcgagcg taaacagaac cgtatttact acagcaagcc acgcagtgaa     240

atgggtgtga cttgtgttgg tcgccagaaa attaaattag gcagcaaacc acttatttga    300

<210> 6
<211> 651
<212> DNA
<213> Enterobacteria phage lambda

<220>
<221> source
<222> 1..651
<223> /mol_type="DNA"
      /organism="Enterobacteria phage lambda"

<400> 6
atgaatacac aactgatggg tgagcgtatt cgcgctcgca gaaaagaact caagattagg      60

caggctgccc ttggcaagat ggttggcgtg tctaatgttg ctatttccca atgggagcga     120

tctgaaactg agcccaatgg cgaaaaccta ttggccttag ccaaggcttt gcagtgctcc     180

cctgattacc tgttgaaagg agaggatagt ctttcaaaca ttgcctatca cagcaggcat     240

gatccaagag gttcgtatcc tctaattagt tgggtaagcg caggatgttg gatggaagct     300

gtagagccat atcataggcg tgcaatagat aactggtacg acacaacggt agattgttct     360

gaagactctt tttggctcga cgttaaaggc gattcaatga ctgccccggc aggactgagt     420

attcctgagg ggatgattat tctcgtcgac ccagaagtcg aaccacgtaa tggaaagctg     480

gtagtcgcca aacttgaagg agaaaacgag gcgacattca aaaagttagt tattgatgcc     540

ggtagaaaat tcctgaaacc actcaatcca caatacccaa tgattgaaat caatgggaac     600

tgtaaaatca ttggcgttgt cgttgatgcc aagctagcaa accttcctta a              651

<210> 7
<211> 1071
<212> DNA
<213> Enterobacteria phage lambda

<220>
<221> source
<222> 1..1071
<223> /mol_type="DNA"
      /organism="Enterobacteria phage lambda"

<400> 7
atgggaagaa ggcgaagtca tgagcgccgg gatttacccc ctaaccttta tataagaaac      60

aatggatatt actgctacag ggacccaagg acgggtaaag agtttggatt aggcagagac     120

aggcgaatcg caatcactga agctatacag gccaacattg agttattttc aggacacaaa     180

cacaagcctc tgacagcgag aatcaacagt gataattccg ttacgttaca ttcatggctt     240

gatcgctacg aaaaaatcct ggccagcaga ggaatcaagc agaagacact cataaattac     300

atgagcaaaa ttaaagcaat aaggaggggt ctgcctgatg ctccacttga agacatcacc     360

acaaaagaaa ttgcggcaat gctcaatgga tacatagacg agggcaaggc ggcgtcagcc     420

aagttaatca gatcaacact gagcgatgca ttccgagagg caatagctga aggccatata     480

acaacaaacc atgtcgctgc cactcgcgca gcaaaatcag aggtaaggag atcaagactt     540

acggctgacg aatacctgaa aatttatcaa gcagcagaat catcaccatg ttggctcaga 600

cttgcaatgg aactggctgt tgttaccggg caacgagttg gtgatttatg cgaaatgaag 660

tggtctgata tcgtagatgg atatctttat gtcgagcaaa gcaaaacagg cgtaaaaatt 720

gccatcccaa cagcattgca tattgatgct ctcggaatat caatgaagga aacacttgat 780

aaatgcaaag agattcttgg cggagaaacc ataattgcat ctactcgtcg cgaaccgctt 840

tcatccggca cagtatcaag gtattttatg cgcgcacgaa aagcatcagg tctttccttc 900

gaaggggatc cgcctacctt tcacgagttg cgcagtttgt ctgcaagact ctatgagaag 960

cagataagcg ataagtttgc tcaacatctt ctcgggcata agtcggacac catggcatca 1020

cagtatcgtg atgacagagg cagggagtgg gacaaaattg aaatcaaata a 1071

<210> 8
<211> 219
<212> DNA
<213> Enterobacteria phage lambda

<220>
<221> source
<222> 1..219
<223> /mol_type="DNA"
/organism="Enterobacteria phage lambda"

<400> 8
atgtacttga cacttcagga gtggaacgca cgccagcgac gtccaagaag ccttgaaaca 60

gttcgtcgat gggttcggga atgcaggata ttcccacctc cggttaagga tggaagagag 120

tatctgttcc acgaatcagc ggtaaaggtt gacttaaatc gaccagtaac aggtggcctt 180

ttgaagagga tcagaaatgg gaagaaggcg aagtcatga 219

<210> 9
<211> 232
<212> DNA
<213> Enterobacteria phage lambda

<220>
<221> source
<222> 1..232
<223> /mol_type="DNA"
/organism="Enterobacteria phage lambda"

<400> 9
tacaggtcac taataccatc taagtagttg attcatagtg actgcatatg ttgtgtttta 60

cagtattatg tagtctgttt tttatgcaaa atctaattta atatattgat atttatatca 120

ttttacgttt ctcgttcagc ttttttatac taagttggca ttataaaaaa gcattgctta 180

tcaatttgtt gcaacgaaca ggtcactatc agtcaaaata aaatcattat tt 232

<210> 10
<211> 29865
<212> DNA
<213> artificial sequences

<220>

<221> source
<222> 1..29865
<223> /mol_type="DNA"
      /note="Genetically modified phage"
      /organism="artificial sequences"

<400> 10
gcttttttat actaagttgg cattataaaa aagcattgct tatcaatttg ttgcaacgaa      60

caggtcacta tcagtcaaaa taaaatcatt atttgatttc aattttgtcc cactccctgc     120

ctctgtcatc acgatactgt gatgccatgg tgtccgactt atgcccgaga agatgttgag     180

caaacttatc gcttatctgc ttctcataga gtcttgcaga caaactgcgc aactcgtgaa     240

aggtaggcgg atccagatcc cggacaccat cgaatggcgc aaaacctttc gcggtatggc     300

atgatagcgc ccggaagaga gtcaattcag ggtggtgaat gtgaaaccag taacgttata     360

cgatgtcgca gagtatgccg gtgtctctta tcagaccgtt tcccgcgtgg tgaaccaggc     420

cagccacgtt tctgcgaaaa cgcgggaaaa agtggaagcg gcgatggcgg agctgaatta     480

cattcccaac cgcgtggcac aacaactggc gggcaaacag tcgttgctga ttggcgttgc     540

cacctccagt ctggccctgc acgcgccgtc gcaaattgtc gcggcgatta aatctcgcgc     600

cgatcaactg ggtgccagcg tggtggtgtc gatggtagaa cgaagcggcg tcgaagcctg     660

taaagcggcg gtgcacaatc ttctcgcgca acgcgtcagt gggctgatca ttaactatcc     720

gctggatgac caggatgcca ttgctgtgga agctgcctgc actaatgttc cggcgttatt     780

tcttgatgtc tctgaccaga cacccatcaa cagtattatt ttctcccatg aagacggtac     840

gcgactgggc gtggagcatc tggtcgcatt gggtcaccag caaatcgcgc tgttagcggg     900

cccattaagt tctgtctcgg cgcgtctgcg tctggctggc tggcataaat atctcactcg     960

caatcaaatt cagccgatag cggaacggga aggcgactgg agtgccatgt ccggttttca    1020

acaaaccatg caaatgctga atgagggcat cgttcccact gcgatgctgg ttgccaacga    1080

tcagatggcg ctgggcgcaa tgcgcgccat taccgagtcc gggctgcgcg ttggtgcgga    1140

tatctcggta gtgggatacg acgataccga agacagctca tgttatatcc cgccgttaac    1200

caccatcaaa caggattttc gcctgctggg gcaaaccagc gtggaccgct tgctgcaact    1260

ctctcagggc caggcggtga agggcaatca gctgttgccc gtctcactgg tgaaaagaaa    1320

aaccaccctg gcgcccaata cgcaaaccgc ctctccccgc gcgttggccg attcattaat    1380

gcagctggca cgacaggttt cccgactgga aagcgggcag tgagcgcaac gcaattaatg    1440

taagttagct cactcattag gcaccccagg ctttacactt tatgcttccg gctcgtataa    1500

tgtgtggaat tgtgagcgga taacaatttc acacaggaaa cagctatgac catgattacg    1560

gattcactgg ccgtcgtttt acaacgtcgt gactgggaaa accctggcgt tacccaactt    1620

aatcgccttg cagcacatcc ccctttcgcc agctggcgta atagcgaaga ggcccgcacc    1680

gatcgccctt cccaacagtt gcgcagcctg aatggcgaat ggcgctttgc ctggtttccg    1740

gcaccagaag cggtgccgga aagctggctg gagtgcgatc ttcctgaggc cgatactgtc    1800

gtcgtcccct caaactggca gatgcacggt tacgatgcgc ccatctacac caacgtgacc    1860

tatcccatta cggtcaatcc gccgtttgtt cccacggaga atccgacggg ttgttactcg 1920
ctcacattta atgttgatga aagctggcta caggaaggcc agacgcgaat tatttttgat 1980
ggcgtcggga tctgatccgg atttactaac tggaagaggc actaaatgaa cacgattaac 2040
atcgctaaga acgacttctc tgacatcgaa ctggctgcta tcccgttcaa cactctggct 2100
gaccattacg gtgagcgttt agctcgcgaa cagttggccc ttgagcatga gtcttacgag 2160
atgggtgaag cacgcttccg caagatgttt gagcgtcaac ttaaagctgg tgaggttgcg 2220
gataacgctg ccgccaagcc tctcatcact accctactcc ctaagatgat tgcacgcatc 2280
aacgactggt ttgaggaagt gaaagctaag cgcggcaagc gcccgacagc cttccagttc 2340
ctgcaagaaa tcaagccgga agccgtagcg tacatcacca ttaagaccac tctggcttgc 2400
ctaaccagtg ctgacaatac aaccgttcag gctgtagcaa gcgcaatcgg tcgggccatt 2460
gaggacgagg ctcgcttcgg tcgtatccgt gaccttgaag ctaagcactt caagaaaaac 2520
gttgaggaac aactcaacaa gcgcgtaggg cacgtctaca agaaagcatt tatgcaagtt 2580
gtcgaggctg acatgctctc taagggtcta ctcggtggcg aggcgtggtc ttcgtggcat 2640
aaggaagact ctattcatgt aggagtacgc tgcatcgaga tgctcattga gtcaaccgga 2700
atggttagct tacaccgcca aaatgctggc gtagtaggtc aagactctga gactatcgaa 2760
ctcgcacctg aatacgctga ggctatcgca acccgtgcag gtgcgctggc tggcatctct 2820
ccgatgttcc aaccttgcgt agttcctcct aagccgtgga ctggcattac tggtggtggc 2880
tattgggcta acggtcgtcg tcctctggcg ctggtgcgta ctcacagtaa gaaagcactg 2940
atgcgctacg aagacgttta catgcctgag gtgtacaaag cgattaacat tgcgcaaaac 3000
accgcatgga aaatcaacaa gaaagtccta gcggtcgcca acgtaatcac caagtggaag 3060
cattgtccgg tcgaggacat ccctgcgatt gagcgtgaag aactcccgat gaaaccggaa 3120
gacatcgaca tgaatcctga ggctctcacc gcgtggaaac gtgctgccgc tgctgtgtac 3180
cgcaaggaca aggctcgcaa gtctcgccgt atcagccttg agttcatgct tgagcaagcc 3240
aataagtttg ctaaccataa ggccatctgg ttcccttaca acatggactg gcgcggtcgt 3300
gtttacgctg tgtcaatgtt caacccgcaa ggtaacgata tgaccaaagg actgcttacg 3360
ctggcgaaag gtaaaccaat cggtaaggaa ggttactact ggctgaaaat ccacggtgca 3420
aactgtgcgg gtgtcgataa ggttccgttc cctgagcgca tcaagttcat tgaggaaaac 3480
cacgagaaca tcatggcttg cgctaagtct ccactggaga acacttggtg ggctgagcaa 3540
gattctccgt tctgcttcct tgcgttctgc tttgagtacg ctggggtaca gcaccacggc 3600
ctgagctata actgctccct tccgctggcg tttgacgggt cttgctctgg catccagcac 3660
ttctccgcga tgctccgaga tgaggtaggt ggtcgcgcgg ttaacttgct tcctagtgaa 3720
accgttcagg acatctacgg gattgttgct aagaaagtca acgagattct acaagcagac 3780
gcaatcaatg ggaccgataa cgaagtagtt accgtgaccg atgagaacac tggtgaaatc 3840
tctgagaaag tcaagctggg cactaaggca ctggctggtc aatggctggc ttacggtgtt 3900

actcgcagtg tgactaagcg ttcagtcatg acgctggctt acgggtccaa agagttcggc 3960
ttccgtcaac aagtgctgga agataccatt cagccagcta ttgattccgg caagggtctg 4020
atgttcactc agccgaatca ggctgctgga tacatggcta agctgatttg ggaatctgtg 4080
agcgtgacgg tggtagctgc ggttgaagca atgaactggc ttaagtctgc tgctaagctg 4140
ctggctgctg aggtcaaaga taagaagact ggagagattc ttcgcaagcg ttgcgctgtg 4200
cattgggtaa ctcctgatgg tttccctgtg tggcaggaat acaagaagcc tattcagacg 4260
cgcttgaacc tgatgttcct cggtcagttc cgcttacagc ctaccattaa caccaacaaa 4320
gatagcgaga ttgatgcaca caaacaggag tctggtatcg ctcctaactt tgtacacagc 4380
caagacggta gccaccttcg taagactgta gtgtgggcac acgagaagta cggaatcgaa 4440
tcttttgcac tgattcacga ctccttcggt accattccgg ctgacgctgc gaacctgttc 4500
aaagcagtgc gcgaaactat ggttgacaca tatgagtctt gtgatgtact ggctgatttc 4560
tacgaccagt tcgctgacca gttgcacgag tctcaattgg acaaaatgcc agcacttccg 4620
gctaaaggta acttgaacct ccgtgacatc ttagagtcgg acttcgcgtt cgcgtaacgc 4680
caaatcaata cgactccgga tccccttcga aggaaagacc tgatgctttt cgtgcgcgca 4740
taaaatacct tgatactgtg ccggatgaaa gcggttcgcg acgagtagat gcaattatgg 4800
tttctccgcc aagaatctct ttgcatttat caagtgtttc cttcattgat attccgagag 4860
catcaatatg caatgctgtt gggatggcaa tttttacgcc tgttttgctt tgctcgacat 4920
aaagatatcc atctacgata tcagaccact tcatttcgca taaatcacca actcgttgcc 4980
cggtaacaac agccagttcc attgcaagtc tgagccaaca tggtgatgat tctgctgctt 5040
gataaatttt caggtattcg tcagccgtaa gtcttgatct ccttacctct gattttgctg 5100
cgcgagtggc agcgacatgg tttgttgtta tatggccttc agctattgcc tctcggaatg 5160
catcgctcag tgttgatctg attaacttgg ctgacgccgc cttgccctcg tctatgtatc 5220
cattgagcat tgccgcaatt tcttttgtgg tgatgtcttc aagtggagca tcaggcagac 5280
ccctccttat tgctttaatt ttgctcatgt aatttatgag tgtcttctgc ttgattcctc 5340
tgctggccag gattttttcg tagcgatcaa gccatgaatg taacgtaacg gaattatcac 5400
tgttgattct cgctgtcaga ggcttgtgtt tgtgtcctga aaataactca atgttggcct 5460
gtatagcttc agtgattgcg attcgcctgt ctctgcctaa tccaaactct ttacccgtcc 5520
ttgggtccct gtagcagtaa tatccattgt ttcttatata aaggttaggg ggtaaatccc 5580
ggcgctcatg acttcgcctt cttcacactg gagggcaaag aagatttcca ataatcagaa 5640
caagtcggct cctgtttagt tacgagcgac attgctccgt gtattcactc gttggaatga 5700
atacacagtg cagtgtttat tctgttattt atgccaaaaa taaaggccac tatcaggcag 5760
ctttgttgtt ctgtttacca agttctctgg caatcattgc cgtcgttcgt attgcccatt 5820
tatcgacata tttcccatct tccattacag gaaacatttc ttcaggctta accatgcatt 5880
ccgattgcag cttgcatcca ttgcatcgct tgaattgtcc acaccattga tttttatcaa 5940

tagtcgtagt catacggata gtcctggtat tgttccatca catcctgagg atgctcttcg 6000
aactcttcaa attcttcttc catatatcac ctcaaataag tggtttgctg cctaatttaa 6060
ttttctggcg accaacacaa gtcacaccca tttcactgcg tggcttgctg tagtaaatac 6120
ggttctgttt acgctcgact tcttctgcct tcttgcagcg aaggcttccg agtgatgctg 6180
ctttatctgc tctgacgcaa ccagagagct ttagcgcaat ttttcgcgcc agtgcttcat 6240
tactgcgtcg ctcggcaata agttctgctc tgcgagcttt gtagcggctt tttgccgtac 6300
ctttggattc tttccagaca atggttacca tgatggtctc ctttaagtgg ctttggcgca 6360
tgacgcgtcg aggtgcttat cttctcgatc gctgtcttgt agctgcaatt cgcgccatcc 6420
ccaaaaccac tcaagttctg gtctcaacgg ttaggttgag agtccgtcga tgttaaagag 6480
cctgccaatc tgttccgttt ggcttccagc gtcctgctga tggcttaaat ttaagacttc 6540
ttaatttatt ggtcaagtgc atttttgaag aaaacttaat tttatgggcg tgaatttagt 6600
ttgtctttga tttttaacgg gaaataaaaa aggggcgaaa gccccttaag gaaggtttgc 6660
tagcttggca tcaacgacaa cgccaatgat tttacagttc ccattgattt caatcattgg 6720
gtattgtgga ttgagtggtt tcaggaattt tctaccggca tcaataacta actttttgaa 6780
tgtcgcctcg ttttctcctt caagtttggc gactaccagc tttccattac gtggttcgac 6840
ttctgggtcg acgagaataa tcatcccctc aggaatactc agtcctgccg gggcagtcat 6900
tgaatcgcct ttaacgtcga gccaaaaaga gtcttcagaa caatctaccg ttgtgtcgta 6960
ccagttatct attgcacgcc tatgatatgg ctctacagct tccatccaac atcctgcgct 7020
tacccaacta attagaggat acgaacctct tggatcatgc ctgctgtgat aggcaatgtt 7080
tgaaagacta tcctctcctt tcaacaggta atcaggggag cactgcaaag ccttggctaa 7140
ggccaatagg ttttcgccat tgggctcagt ttcagatcgc tcccattggg aaatagcaac 7200
attagacacg ccaaccatct tgccaagggc agcctgccta atcttgagtt cttttctgcg 7260
agcgcgaata cgctcaccca tcagttgtgt attcatagtt aagacatctt aaataaactt 7320
gacttaagat tcctttggtg gataatttaa gtgttcttta atttcggagc gagtctatgt 7380
acaaaaaaga tgttattgac cacttcggaa cccagcgtgc tgttgctaaa gcactaggca 7440
ttagcgatgc agcagtctct cagtggaaag aagttatccc agagaaagac gcctatcgat 7500
tggaaatcgt tacagctggc gccctgaagt atcaagaaag tgcttaccgc caagcggcat 7560
aagcaaattg ctctttaaca gttctggcct ttcacctcta accgggtgag caaacatcag 7620
cggcaaatcc attgggtgtg ccgctataac tcaatatcaa tataggtaaa ttaacaaatg 7680
gcacaagcaa gctacagcaa gccaacacag cgagaaattg atcgcgctga aactgattta 7740
ctcatcaacc tgtcaacgct tacccagcgc ggtctggcaa agatgattgg ctgtcatgaa 7800
tcgaagataa gcagaacgga ctggagattt attgcttcgg tcttgtgtgc tttcggaatg 7860
gcatcagaca tcagtccgat tagcagggct tttaagtatg cgcttgatgg actcacaaag 7920
aaaaaacgcc cggtgtgcaa gaccgagcgt tctgaacaaa tccagatgga gttctgaggt 7980

cattactgga tctatcaaca ggagtcatta tgacaaatac agcaaaaata ctcaacttcg 8040
gcagaggtaa ctttgccgga caggagcgta atgtggcaga tctcgatgat ggttacgcca 8100
gactatcaaa tatgctgctt gaggcttatt cgggcgcaga tctgaccaag cgacagttta 8160
aagtgctgct tgccattctg cgtaaaacct atgggtggaa taaaccaatg gacagaatca 8220
ccgattctca acttagcgag attacaaagt tacctgtcaa acggtgcaat gaagccaagt 8280
tagaactcgt cagaatgaat attatcaagc agcaaggcgg catgtttgga ccaaataaaa 8340
acatctcaga atggtgtatc cctcaaaacg agggaaaatc ccctaaaacg agggataaaa 8400
catccctcaa attgggggat tgctatccct caaaacaggg ggacacaaaa gacactatta 8460
caaaagaaaa aagaaaagat tattcgtcag agaattctgg cgaatcctct gaccagccag 8520
aaaacgacct ttctgtggtg aaaccggatg ctgcaattca gagcggcagc aagtggggga 8580
cagcagaaga cctgaccgcc gcagagtgga tgtttgacat ggtgaagact atcgcaccat 8640
cagccagaaa accgaatttt gctgggtggg ctaacgatat ccgcctgatg cgtgaacgtg 8700
acggacgtaa ccaccgcgac atgtgtgtgc tgttccgctg ggcatgccag gacaacttct 8760
ggtccggtaa cgtgctgagc ccggccaaac tccgcgataa gtggacccaa ctcgaaatca 8820
accgtaacaa gcaacaggca ggcgtgacag ccagcaaacc aaaactcgac ctgacaaaca 8880
cagactggat ttacggggtg gatctatgaa aaacatcgcc gcacagatgg ttaactttga 8940
ccgtgagcag atgcgtcgga tcgccaacaa catgccggaa cagtacgacg aaaagccgca 9000
ggtacagcag gtagcgcaga tcatcaacgg tgtgttcagc cagttactgg caactttccc 9060
ggcgagcctg gctaaccgtg accagaacga agtgaacgaa atccgtcgcc agtgggttct 9120
ggcttttcgg gaaaacggga tcaccacgat ggaacaggtt aacgcaggaa tgcgcgtagc 9180
ccgtcggcag aatcgaccat ttctgccatc acccgggcag tttgttgcat ggtgccggga 9240
agaagcatcc gttaccgccg gactgccaaa cgtcagcgag ctggttgata tggtttacga 9300
gtattgccgg aagcgaggcc tgtatccgga tgcggagtct tatccgtgga aatcaaacgc 9360
gcactactgg ctggttacca acctgtatca gaacatgcgg gccaatgcgc ttactgatgc 9420
ggaattacgc cgtaaggccg cagatgagct tgtccatatg actgcgagaa ttaaccgtgg 9480
tgaggcgatc cctgaaccag taaaacaact tcctgtcatg ggcggtagac ctctaaatcg 9540
tgcacaggct ctggcgaaga tcgcagaaat caaagctaag ttcggactga aaggagcaag 9600
tgtatgacgg gcaaagaggc aattattcat tacctgggga cgcataatag cttctgtgcg 9660
ccggacgttg ccgcgctaac aggcgcaaca gtaaccagca taaatcaggc cgcggctaaa 9720
atggcacggg caggtcttct ggttatcgaa ggtaaggtct ggcgaacggt gtattaccgg 9780
tttgctacca gggaagaacg ggaaggaaag atgagcacga acctgatgaa caaactggat 9840
acgattggat tcgacaacaa aaaagacctg cttatctcgg tgggcgattt ggttgatcgt 9900
ggtgcagaga acgttgaatg cctggaatta atcacattcc cctggttcag agctgtacgt 9960
ggaaaccatg agcaaatgat gattgatggc ttatcagagc gtggaaacgt taatcactgg 10020

ctgcttaatg gcggtggctg gttctttaat ctcgattacg acaaagaaat tctggctaaa 10080
gctcttgccc ataaagcaga tgaacttccg ttaatcatcg aactggtgag caaagataaa 10140
aaatatgtta tctgccacgc cgattatccc tttgacgaat acgagtttgg aaagccagtt 10200
gatcatcagc aggtaatctg gaaccgcgaa cgaatcagca actcacaaaa cgggatcgtg 10260
aaagaaatca aaggcgcgga cacgttcatc tttggtcata cgccagcagt gaaaccactc 10320
aagtttgcca accaaatgta tatcgatacc ggcgcagtgt tctgcggaaa cctaacattg 10380
attcaggtac agggagaagg cgcatgagac tcgaaagcgt agctaaattt cattcgccaa 10440
aaagcccgat gatgagcgac tcaccacggg ccacggcttc tgactctctt tccggtactg 10500
atgtgatggc tgctatgggg atggcgcaat cacaagccgg attcggtatg gctgcattct 10560
gcggtaagca cgaactcagc cagaacgaca aacaaaaggc tatcaactat ctgatgcaat 10620
ttgcacacaa ggtatcgggg aaataccgtg gtgtggcata tcttgaagga aatactaagg 10680
caaaggtact gcaagtgctc gcaacattcg cttatgcgga ttattgccgt agtgccgcga 10740
cgccgggggc aagatgcaga gattgccatg gtacaggccg tgcggttgat attgccaaaa 10800
cagagctgtg ggggagagtt gtcgagaaag agtgcggaag atgcaaaggc gtcggctatt 10860
caaggatgcc agcaagcgca gcatatcgcg ctgtgacgat gctaatccca aaccttaccc 10920
aacccacctg gtcacgcact gttaagccgc tgtatgacgc tctggtggtg caatgccaca 10980
aagaagagtc aatcgcagac aacattttga atgcggtcac acgttagcag catgattgcc 11040
acggatggca acatattaac ggcatgatat tgacttattg aataaaattg ggtaaatttg 11100
actcaacgat gggttaattc gctcgttgtg gtagtgagat gaaaagaggc ggcgcttact 11160
accgattccg cctagttggt cacttcgacg tatcgtctgg aactccaacc atcgcaggca 11220
gagaggtctg caaaatgcaa tcccgaaaca gttcgcaggt aatagttaga gcctgcataa 11280
cggtttcggg attttttata tctgcacaac aggtaagagc attgagtcga taatcgtgaa 11340
gagtcggcga gcctggttag ccagtgctct ttccgttgtg ctgaattaag cgaataccgg 11400
aagcagaacc ggatcaccaa atgcgtacag gcgtcatcgc cgcccagcaa cagcacaacc 11460
caaactgagc cgtagccact gtctgtcctg aattccatgc ttgaacccgc ctatgcgcgg 11520
gttttctttt gtgcgcttgc aggccagctt gggatcagca gcctgacgga tgcggtgtcc 11580
ggcgacagcc tgactgccca ggaggcactc gcgacgctgg cattatccgg tgatgatgac 11640
ggaccacgac aggcccgcag ttatcaggtc atgaacggca tcgccgtgct gccggtgtcc 11700
ggcacgctgg tcagccggac gcgggcgctg cagccgtact cggggatgac cggttacaac 11760
ggcattatcg cccgtctgca acaggctgcc agcgatccga tggtggacgg cattctgctc 11820
gatatggaca cgcccggcgg gatggtggcg ggggcatttg actgcgctga catcatcgcc 11880
cgtgtgcgtg acataaaacc ggtatgggcg cttgccaacg acatgaactg cagtgcaggt 11940
cagttgcttg ccagtgccgc ctcccggcgt ctggtcacgc agaccgcccg gacaggctcc 12000
atcggcgtca tgatggctca cagtaattac ggtgctgcgc tggagaaaca gggtgtggaa 12060

atcacgctga tttacagcgg cagccataag gtggatggca acccctacag ccatcttccg 12120
gatgacgtcc gggagacact gcagtcccgg atggacgcaa cccgccagat gtttgcgcag 12180
aaggtgtcgg catataccgg cctgtccgtg caggttgtgc tggataccga ggctgcagtg 12240
tacagcggtc aggaggccat tgatgccgga ctggctgatg aacttgttaa cagcaccgat 12300
gcgatcaccg tcatgcgtga tgcactggat gcacgtaaat cccgtctctc aggagggcga 12360
atgaccaaag agactcaatc aacaactgtt tcagccactg cttcgcaggc tgacgttact 12420
gacgtggtgc cagcgacgga gggcgagaac gccagcgcgg cgcagccgga cgtgaacgcg 12480
cagatcaccg cagcggttgc ggcagaaaac agccgcatta tggggatact caactgtgag 12540
gaggctcacg gacgcgaaga acaggcacgc gtgctggcag aaacccccgg tatgaccgtg 12600
aaaacggccc gccgcattct ggccgcagca ccacagagtg cacaggcgcg cagtgacact 12660
gcgctggatc gtctgatgca gggggcaccg gcaccgctgg ctgcaggtaa cccggcatct 12720
gatgccgtta acgatttgct gaacacacca gtgtaaggga tgtttatgac gagcaaagaa 12780
acctttaccc attaccagcc gcagggcaac agtgacccgg ctcataccgc aaccgcgccc 12840
ggcggattga gtgcgaaagc gcctgcaatg accccgctga tgctggacac ctccagccgt 12900
aagctggttg cgtgggatgg caccaccgac ggtgctgccg ttggcattct tgcggttgct 12960
gctgaccaga ccagcaccac gctgacgttc tacaagtccg gcacgttccg ttatgaggat 13020
gtgctctggc cggaggctgc cagcgacgag acgaaaaaac ggaccgcgtt tgccggaacg 13080
gcaatcagca tcgtttaact ttacccttca tcactaaagg ccgcctgtgc ggcttttttt 13140
acgggatttt tttatgtcga tgtacacaac cgcccaactg ctggcggcaa atgagcagaa 13200
atttaagttt gatccgctgt ttctgcgtct ctttttccgt gagagctatc ccttcaccac 13260
ggagaaagtc tatctctcac aaattccggg actggtaaac atggcgctgt acgtttcgcc 13320
gattgtttcc ggtgaggtta tccgttcccg tggcggctcc acctctgaat ttacgccggg 13380
atatgtcaag ccgaagcatg aagtgaatcc gcagatgacc ctgcgtcgcc tgccggatga 13440
agatccgcag aatctggcgg acccggctta ccgccgccgt cgcatcatca tgcagaacat 13500
gcgtgacgaa gagctggcca ttgctcaggt cgaagagatg caggcagttt ctgccgtgct 13560
taagggcaaa tacaccatga ccggtgaagc cttcgatccg gttgaggtgg atatgggccg 13620
cagtgaggag aataacatca cgcagtccgg cggcacggag tggagcaagc gtgacaagtc 13680
cacgtatgac ccgaccgacg atatcgaagc ctacgcgctg aacgccagcg gtgtggtgaa 13740
tatcatcgtg ttcgatccga aaggctgggc gctgttccgt tccttcaaag ccgtcaagga 13800
gaagctggat acccgtcgtg gctctaattc cgagctggag acagcggtga aagacctggg 13860
caaagcggtg tcctataagg ggatgtatgg cgatgtggcc atcgtcgtgt attccggaca 13920
gtacgtggaa aacggcgtca aaaagaactt cctgccggac aacacgatgg tgctggggaa 13980
cactcaggca cgcggtctgc gcacctatgg ctgcattcag gatgcggacg cacagcgcga 14040
aggcattaac gcctctgccc gttacccgaa aaactgggtg accaccggcg atccggcgcg 14100

tgagttcacc atgattcagt cagcaccgct gatgctgctg gctgaccctg atgagttcgt 14160
gtccgtacaa ctggcgtaat catggccctt cggggccatt gtttctctgt ggaggagtcc 14220
atgacgaaag atgaactgat tgcccgtctc cgctcgctgg gtgaacaact gaaccgtgat 14280
gtcagcctga cggggacgaa agaagaactg gcgctccgtg tggcagagct gaaagaggag 14340
cttgatgaca cggatgaaac tgccggtcag gacacccctc tcagccggga aaatgtgctg 14400
accggacatg aaaatgaggt gggatcagcg cagccggata ccgtgattct ggatacgtct 14460
gaactggtca cggtcgtggc actggtgaag ctgcatactg atgcacttca cgccacgcgg 14520
gatgaacctg tggcatttgt gctgccggga acggcgtttc gtgtctctgc cggtgtggca 14580
gccgaaatga cagagcgcgg cctggccaga atgcaataac gggaggcgct gtggctgatt 14640
tcgataacct gttcgatgct gccattgccc gcgccgatga aacgatacgc gggtacatgg 14700
gaacgtcagc caccattaca tccggtgagc agtcaggtgc ggtgatacgt ggtgtttttg 14760
atgaccctga aaatatcagc tatgccggac agggcgtgcg cgttgaaggc tccagcccgt 14820
ccctgtttgt ccggactgat gaggtgcggc agctgcggcg tggagacacg ctgaccatcg 14880
gtgaggaaaa tttctgggta gatcgggttt cgccggatga tggcggaagt tgtcatctct 14940
ggcttggacg gggcgtaccg cctgccgtta accgtcgccg ctgaaagggg gatgtatggc 15000
cataaaaggt cttgagcagg ccgttgaaaa cctcagccgt atcagcaaaa cggcggtgcc 15060
tggtgccgcc gcaatggcca ttaaccgcgt tgcttcatcc gcgatatcgc agtcggcgtc 15120
acaggttgcc cgtgagacaa aggtacgccg gaaactggta aaggaaaggg ccaggctgaa 15180
aagggccacg gtcaaaaatc cgcaggccag aatcaaagtt aaccgggggg atttgcccgt 15240
aatcaagctg ggtaatgcgc gggttgtcct ttcgcgccgc aggcgtcgta aaaaggggca 15300
gcgttcatcc ctgaaaggtg gcggcagcgt gcttgtggtg ggtaaccgtc gtattcccgg 15360
cgcgtttatt cagcaactga aaaatggccg gtggcatgtc atgcagcgtg tggctgggaa 15420
aaaccgttac cccattgatg tggtgaaaat cccgatggcg gtgccgctga ccacggcgtt 15480
taaacaaaat attgagcgga tacggcgtga acgtcttccg aaagagctgg gctatgcgct 15540
gcagcatcaa ctgaggatgg taataaagcg atgaaacata ctgaactccg tgcagccgta 15600
ctggatgcac tggagaagca tgacaccggg gcgacgtttt ttgatggtcg ccccgctgtt 15660
tttgatgagg cggattttcc ggcagttgcc gtttatctca ccggcgctga atacacgggc 15720
gaagagctgg acagcgatac ctggcaggcg gagctgcata tcgaagtttt cctgcctgct 15780
caggtgccgg attcagagct ggatgcgtgg atggagtccc ggatttatcc ggtgatgagc 15840
gatatcccgg cactgtcaga tttgatcacc agtatggtgg ccagcggcta tgactaccgg 15900
cgcgacgatg atgcgggctt gtggagttca gccgatctga cttatgtcat tacctatgaa 15960
atgtgaggac gctatgcctg taccaaatcc tacaatgccg gtgaaaggtg ccgggaccac 16020
cctgtgggtt tataagggga gcggtgaccc ttacgcgaat ccgctttcag acgttgactg 16080
gtcgcgtctg gcaaaagtta aagacctgac gcccggcgaa ctgaccgctg agtcctatga 16140

cgacagctat ctcgatgatg aagatgcaga ctggactgcg accgggcagg ggcagaaatc 16200
tgccggagat accagcttca cgctggcgtg gatgcccgga gagcaggggc agcaggcgct 16260
gctggcgtgg tttaatgaag gcgatacccg tgcctataaa atccgcttcc cgaacggcac 16320
ggtcgatgtg ttccgtggct gggtcagcag tatcggtaag gcggtgacgg cgaaggaagt 16380
gatcacccgc acggtgaaag tcaccaatgt gggacgtccg tcgatggcag aagatcgcag 16440
cacggtaaca gcggcaaccg gcatgaccgt gacgcctgcc agcacctcgg tggtgaaagg 16500
gcagagcacc acgctgaccg tggccttcca gccggagggc gtaaccgaca agagctttcg 16560
tgcggtgtct gcggataaaa caaaagccac cgtgtcggtc agtggtatga ccatcaccgt 16620
gaacggcgtt gctgcaggca aggtcaacat tccggttgta tccggtaatg gtgagtttgc 16680
tgcggttgca gaaattaccg tcaccgccag ttaatccgga gagtcagcga tgttcctgaa 16740
aaccgaatca tttgaacata acggtgtgac cgtcacgctt tctgaactgt cagccctgca 16800
gcgcattgag catctcgccc tgatgaaacg gcaggcagaa caggcggagt cagacagcaa 16860
ccggaagttt actgtggaag acgccatcag aaccggcgcg tttctggtgg cgatgtccct 16920
gtggcataac catccgcaga agacgcagat gccgtccatg aatgaagccg ttaaacagat 16980
tgagcaggaa gtgcttacca cctggcccac ggaggcaatt tctcatgctg aaaacgtggt 17040
gtaccggctg tctggtatgt atgagtttgt ggtgaataat gcccctgaac agacagagga 17100
cgccgggccc gcagagcctg tttctgcggg aaagtgttcg acggtgagct gagttttgcc 17160
ctgaaactgg cgcgtgagat ggggcgaccc gactggcgtg ccatgcttgc cgggatgtca 17220
tccacggagt atgccgactg gcaccgcttt tacagtaccc attattttca tgatgttctg 17280
ctggatatgc acttttccgg gctgacgtac accgtgctca gcctgttttt cagcgatccg 17340
gatatgcatc cgctggattt cagtctgctg aaccggcgcg aggctgacga agagcctgaa 17400
gatgatgtgc tgatgcagaa agcggcaggg cttgccggag gtgtccgctt tggcccggac 17460
gggaatgaag ttatccccgc ttccccggat gtggcggaca tgacggagga tgacgtaatg 17520
ctgatgacag tatcagaagg gatcgcagga ggagtccggt atggctgaac cggtaggcga 17580
tctggtcgtt gatttgagtc tggatgcggc cagatttgac gagcagatgg ccagagtcag 17640
gcgtcatttt tctggtacgg aaagtgatgc gaaaaaaaca gcggcagtcg ttgaacagtc 17700
gctgagccga caggcgctgg ctgcacagaa agcggggatt tccgtcgggc agtataaagc 17760
cgccatgcgt atgctgcctg cacagttcac cgacgtggcc acgcagcttg caggcgggca 17820
aagtccgtgg ctgatcctgc tgcaacaggg ggggcaggtg aaggactcct tcggcgggat 17880
gatccccatg ttcagggggc ttgccggtgc gatcaccctg ccgatggtgg gggccacctc 17940
gctggcggtg gcgaccggtg cgctggcgta tgcctggtat cagggcaact caaccctgtc 18000
cgatttcaac aaaacgctgg tcctttccgg caatcaggcg ggactgacgg cagatcgtat 18060
gctggtcctg tccagagccg ggcaggcggc agggctgacg tttaaccaga ccagcgagtc 18120
actcagcgca ctggttaagg cgggggtaag cggtgaggct cagattgcgt ccatcagcca 18180

gagtgtggcg cgtttctcct ctgcatccgg cgtggaggtg gacaaggtcg ctgaagcctt 18240
cgggaagctg accacagacc cgacgtcggg gctgacggcg atggctcgcc agttccataa 18300
cgtgtcggcg gagcagattg cgtatgttgc tcagttgcag cgttccggcg atgaagccgg 18360
ggcattgcag gcggcgaacg aggccgcaac gaaagggttt gatgaccaga cccgccgcct 18420
gaaagagaac atgggcacgc tggagacctg ggcagacagg actgcgcggg cattcaaatc 18480
catgtgggat gcggtgctgg atattggtcg tcctgatacc gcgcaggaga tgctgattaa 18540
ggcagaggct gcgtataaga aagcagacga catctggaat ctgcgcaagg atgattattt 18600
tgttaacgat gaagcgcggg cgcgttactg ggatgatcgt gaaaaggccc gtcttgcgct 18660
tgaagccgcc cgaaagaagg ctgagcagca gactcaacag gacaaaaatg cgcagcagca 18720
gagcgatacc gaagcgtcac ggctgaaata taccgaagag gcgcagaagg cttacgaacg 18780
gctgcagacg ccgctggaga aatataccgc ccgtcaggaa gaactgaaca aggcactgaa 18840
agacgggaaa atcctgcagg cggattacaa cacgctgatg gcggcggcga aaaaggatta 18900
tgaagcgacg ctgaaaaagc cgaaacagtc cagcgtgaag gtgtctgcgg gcgatcgtca 18960
ggaagacagt gctcatgctg ccctgctgac gcttcaggca gaactccgga cgctggagaa 19020
gcatgccgga gcaaatgaga aaatcagcca gcagcgccgg gatttgtgga aggcggagag 19080
tcagttcgcg gtactggagg aggcggcgca acgtcgccag ctgtctgcac aggagaaatc 19140
cctgctggcg cataaagatg agacgctgga gtacaaacgc cagctggctg cacttggcga 19200
caaggttacg tatcaggagc gcctgaacgc gctggcgcag caggcggata aattcgcaca 19260
gcagcaacgg gcaaaacggg ccgccattga tgcgaaaagc cgggggctga ctgaccggca 19320
ggcagaacgg gaagccacgg aacagcgcct gaaggaacag tatggcgata atccgctggc 19380
gctgaataac gtcatgtcag agcagaaaaa gacctgggcg gctgaagacc agcttcgcgg 19440
gaactggatg gcaggcctga agtccggctg gagtgagtgg gaagagagcg ccacggacag 19500
tatgtcgcag gtaaaaagtg cagccacgca gacctttgat ggtattgcac agaatatggc 19560
ggcgatgctg accggcagtg agcagaactg gcgcagcttc acccgttccg tgctgtccat 19620
gatgacagaa attctgctta agcaggcaat ggtggggatt gtcgggagta tcggcagcgc 19680
cattggcggg gctgttggtg gcggcgcatc cgcgtcaggc ggtacagcca ttcaggccgc 19740
tgcggcgaaa ttccattttg caaccggagg atttacggga accggcggca aatatgagcc 19800
agcggggatt gttcaccgtg gtgagtttgt cttcacgaag gaggcaacca gccggattgg 19860
cgtggggaat ctttaccggc tgatgcgcgg ctatgccacc ggcggttatg tcggtacacc 19920
gggcagcatg gcagacagcc ggtcgcaggc gtccgggacg tttgagcaga ataaccatgt 19980
ggtgattaac aacgacggca cgaacgggca gataggtccg gctgctctga aggcggtgta 20040
tgacatggcc cgcaagggtg cccgtgatga aattcagaca cagatgcgtg atggtggcct 20100
gttctccgga ggtggacgat gaagaccttc cgctggaaag tgaaacccgg tatggatgtg 20160
gcttcggtcc cttctgtaag aaaggtgcgc tttggtgatg gctattctca gcgagcgcct 20220

gccgggctga atgccaacct gaaaacgtac agcgtgacgc tttctgtccc ccgtgaggag 20280
gccacggtac tggagtcgtt tctggaagag cacgggggct ggaaatcctt tctgtggacg 20340
ccgccttatg agtggcggca gataaaggtg acctgcgcaa aatggtcgtc gcgggtcagt 20400
atgctgcgtg ttgagttcag cgcagagttt gaacaggtgg tgaactgatg caggatatcc 20460
ggcaggaaac actgaatgaa tgcacccgtg cggagcagtc ggccagcgtg gtgctctggg 20520
aaatcgacct gacagaggtc ggtggagaac gttatttttt ctgtaatgag cagaacgaaa 20580
aaggtgagcc ggtcacctgg caggggcgac agtatcagcc gtatcccatt caggggagcg 20640
gttttgaact gaatggcaaa ggcaccagta cgcgccccac gctgacggtt tctaacctgt 20700
acggtatggt caccgggatg gcggaagata tgcagagtct ggtcggcgga acggtggtcc 20760
ggcgtaaggt ttacgcccgt tttctggatg cggtgaactt cgtcaacgga aacagttacg 20820
ccgatccgga gcaggaggtg atcagccgct ggcgcattga gcagtgcagc gaactgagcg 20880
cggtgagtgc ctcctttgta ctgtccacgc cgacggaaac ggatggcgct gtttttccgg 20940
gacgtatcat gctggccaac acctgcacct ggacctatcg cggtgacgag tgcggttata 21000
gcggtccggc tgtcgcggat gaatatgacc agccaacgtc cgatatcacg aaggataaat 21060
gcagcaaatg cctgagcggt tgtaagttcc gcaataacgt cggcaacttt ggcggcttcc 21120
tttccattaa caaactttcg cagtaaatcc catgacacag acagaatcag cgattctggc 21180
gcacgcccgg cgatgtgcgc cagcggagtc gtgcggcttc gtggtaagca cgccggaggg 21240
ggaaagatat ttcccctgcg tgaatatctc cggtgagccg gaggcgtatt tccgtatgtc 21300
gccggaagac tggctgcagg cagaaatgca gggtgagatt gtggcgctgg tccacagcca 21360
ccccggtggt ctgccctggc tgagtgaggc cgaccggcgg ctgcaggtgc agagtgattt 21420
gccgtggtgg ctggtctgcc gggggacgat tcataagttc cgctgtgtgc cgcatctcac 21480
cgggcggcgc tttgagcacg gtgtgacgga ctgttacaca ctgttccggg atgcttatca 21540
tctggcgggg attgagatgc cggactttca tcgtgaggat gactggtggc gtaacggcca 21600
gaatctctat ctggataatc tggaggcgac ggggctgtat caggtgccgt tgtcagcggc 21660
acagccgggc gatgtgctgc tgtgctgttt tggttcatca gtgccgaatc acgccgcaat 21720
ttactgcggc gacggcgagc tgctgcacca tattcctgaa caactgagca aacgagagag 21780
gtacaccgac aaatggcagc gacgcacaca ctccctctgg cgtcaccggg catggcgcgc 21840
atctgccttt acggggattt acaacgattt ggtcgccgca tcgaccttcg tgtgaaaacg 21900
ggggctgaag ccatccgggc actggccaca cagctcccgg cgtttcgtca gaaactgagc 21960
gacggctggt atcaggtacg gattgccggg cgggacgtca gcacgtccgg gttaacggcg 22020
cagttacatg agactctgcc tgatggcgct gtaattcata ttgttcccag agtcgccggg 22080
gccaagtcag gtggcgtatt ccagattgtc ctgggggctg ccgccattgc cggatcattc 22140
tttaccgccg gagccaccct tgcagcatgg ggggcagcca ttggggccgg tggtatgacc 22200
ggcatcctgt tttctctcgg tgccagtatg gtgctcggtg gtgtggcgca gatgctggca 22260

ccgaaagcca gaactccccg tatacagaca acggataacg gtaagcagaa cacctatttc 22320
tcctcactgg ataacatggt tgcccagggc aatgttctgc ctgttctgta cggggaaatg 22380
cgcgtggggt cacgcgtggt ttctcaggag atcagcacgg cagacgaagg ggacggtggt 22440
caggttgtgg tgattggtcg ctgatgcaaa atgttttatg tgaaaccgcc tgcgggcggt 22500
tttgtcattt atggagcgtg aggaatgggt aaaggaagca gtaaggggca taccccgcgc 22560
gaagcgaagg acaacctgaa gtccacgcag ttgctgagtg tgatcgatgc catcagcgaa 22620
gggccgattg aaggtccggt ggatggctta aaaagcgtgc tgctgaacag tacgccggtg 22680
ctggacactg aggggaatac caacatatcc ggtgtcacgg tggtgttccg ggctggtgag 22740
caggagcaga ctccgccgga gggatttgaa tcctccggct ccgagacggt gctgggtacg 22800
gaagtgaaat atgacacgcc gatcacccgc accattacgt ctgcaaacat cgaccgtctg 22860
cgctttacct tcggtgtaca ggcactggtg gaaaccacct caaagggtga caggaatccg 22920
tcggaagtcc gcctgctggt tcagatacaa cgtaacggtg gctgggtgac ggaaaaagac 22980
atcaccatta agggcaaaac cacctcgcag tatctggcct cggtggtgat gggtaacctg 23040
ccgccgcgcc cgtttaatat ccggatgcgc aggatgacgc cggacagcac cacagaccag 23100
ctgcagaaca aaacgctctg gtcgtcatac actgaaatca tcgatgtgaa acagtgctac 23160
ccgaacacgg cactggtcgg cgtgcaggtg gactcggagc agttcggcag ccagcaggtg 23220
agccgtaatt atcatctgcg cgggcgtatt ctgcaggtgc cgtcgaacta taacccgcag 23280
acgcggcaat acagcggtat ctgggacgga acgtttaaac cggcatacag caacaacatg 23340
gcctggtgtc tgtgggatat gctgacccat ccgcgctacg gcatggggaa acgtcttggt 23400
gcggcggatg tggataaatg ggcgctgtat gtcatcggcc agtactgcga ccagtcagtg 23460
ccggacggct ttggcggcac ggagccgcgc atcacctgta atgcgtacct gaccacacag 23520
cgtaaggcgt gggatgtgct cagcgatttc tgctcggcga tgcgctgtat gccggtatgg 23580
aacgggcaga cgctgacgtt cgtgcaggac cgaccgtcgg ataagacgtg gacctataac 23640
cgcagtaatg tggtgatgcc ggatgatggc gcgccgttcc gctacagctt cagcgccctg 23700
aaggaccgcc ataatgccgt tgaggtgaac tggattgacc cgaacaacgg ctgggagacg 23760
gcgacagagc ttgttgaaga tacgcaggcc attgcccgtt acggtcgtaa tgttacgaag 23820
atggatgcct ttggctgtac cagccggggg caggcacacc gcgccgggct gtggctgatt 23880
aaaacagaac tgctggaaac gcagaccgtg gatttcagcg tcggcgcaga agggcttcgc 23940
catgtaccgg gcgatgttat tgaaatctgc gatgatgact atgccggtat cagcaccggt 24000
ggtcgtgtgc tggcggtgaa cagccagacc cggacgctga cgctcgaccg tgaaatcacg 24060
ctgccatcct ccggtaccgc gctgataagc ctggttgacg gaagtggcaa tccggtcagc 24120
gtggaggttc agtccgtcac cgacggcgtg aaggtaaaag tgagccgtgt tcctgacggt 24180
gttgctgaat acagcgtatg ggagctgaag ctgccgacgc tgcgccagcg actgttccgc 24240
tgcgtgagta tccgtgagaa cgacgacggc acgtatgcca tcaccgccgt gcagcatgtg 24300

ccggaaaaag aggccatcgt ggataacggg gcgcactttg acggcgaaca gagtggcacg 24360
gtgaatggtg tcacgccgcc agcggtgcag cacctgaccg cagaagtcac tgcagacagc 24420
ggggaatatc aggtgctggc gcgatgggac acaccgaagg tggtgaaggg cgtgagtttc 24480
ctgctccgtc tgaccgtaac agcggacgac ggcagtgagc ggctggtcag cacggcccgg 24540
acgacggaaa ccacataccg cttcacgcaa ctggcgctgg ggaactacag gctgacagtc 24600
cgggcggtaa atgcgtgggg gcagcagggc gatccggcgt cggtatcgtt ccggattgcc 24660
gcaccggcag caccgtcgag gattgagctg acgccgggct attttcagat aaccgccacg 24720
ccgcatcttg ccgtttatga cccgacggta cagtttgagt tctggttctc ggaaaagcag 24780
attgcggata tcagacaggt tgaaaccagc acgcgttatc ttggtacggc gctgtactgg 24840
atagccgcca gtatcaatat caaaccgggc catgattatt acttttatat ccgcagtgtg 24900
aacaccgttg gcaaatcggc attcgtggag gccgtcggtc gggcgagcga tgatgcggaa 24960
ggttacctgg attttttcaa aggcaagata accgaatccc atctcggcaa ggagctgctg 25020
gaaaaagtcg agctgacgga ggataacgcc agcagactgg aggagttttc gaaagagtgg 25080
aaggatgcca gtgataagtg gaatgccatg tgggctgtca aaattgagca gaccaaagac 25140
ggcaaacatt atgtcgcggg tattggcctc agcatggagg acacggagga aggcaaactg 25200
agccagtttc tggttgccgc caatcgtatc gcatttattg acccggcaaa cgggaatgaa 25260
acgccgatgt ttgtggcgca gggcaaccag atattcatga acgacgtgtt cctgaagcgc 25320
ctgacggccc ccaccattac cagcggcggc aatcctccgg ccttttccct gacaccggac 25380
ggaaagctga ccgctaaaaa tgcggatatc agtggcagtg tgaatgcgaa ctccgggacg 25440
ctcagtaatg tgacgatagc tgaaaactgt acgataaacg gtacgctgag ggcggaaaaa 25500
atcgtcgggg acattgtaaa ggcggcgagc gcggcttttc cgcgccagcg tgaaagcagt 25560
gtggactggc cgtcaggtac ccgtactgtc accgtgaccg atgaccatcc ttttgatcgc 25620
cagatagtgg tgcttccgct gacgtttcgc ggaagtaagc gtactgtcag cggcaggaca 25680
acgtattcga tgtgttatct gaaagtactg atgaacggtg cggtgattta tgatggcgcg 25740
gcgaacgagg cggtacaggt gttctcccgt attgttgaca tgccagcggg tcggggaaac 25800
gtgatcctga cgttcacgct tacgtccaca cggcattcgg cagatattcc gccgtatacg 25860
tttgccagcg atgtgcaggt tatggtgatt aagaaacagg cgctgggcat cagcgtggtc 25920
tgagtgtgtt acagaggttc gtccgggaac gggcgtttta ttataaaaca gtgagaggtg 25980
aacgatgcgt aatgtgtgta ttgccgttgc tgtctttgcc gcacttgcgg tgacagtcac 26040
tccggcccgt gcggaaggtg gacatggtac gtttacggtg ggctattttc aagtgaaacc 26100
gggtacattg ccgtcgttgt cgggcgggga taccggtgtg agtcatctga aagggattaa 26160
cgtgaagtac cgttatgagc tgacggacag tgtgggggtg atggcttccc tggggttcgc 26220
cgcgtcgaaa aagagcagca cagtgatgac cggggaggat acgtttcact atgagagcct 26280
gcgtggacgt tatgtgagcg tgatggccgg accggtttta caaatcagta agcaggtcag 26340

tgcgtacgcc atggccggag tggctcacag tcggtggtcc ggcagtacaa tggattaccg 26400
taagacggaa atcactcccg ggtatatgaa agagacgacc actgccaggg acgaaagtgc 26460
aatgcggcat acctcagtgg cgtggagtgc aggtatacag attaatccgg cagcgtccgt 26520
cgttgttgat attgcttatg aaggctccgg cagtggcgac tggcgtactg acggattcat 26580
cgttggggtc ggttataaat tctgattagc caggtaacac agtgttatga cagcccgccg 26640
gaaccggtgg gcttttttgt ggggtgaata tggcagtaaa gatttcagga gtcctgaaag 26700
acggcacagg aaaaccggta cagaactgca ccattcagct gaaagccaga cgtaacagca 26760
ccacggtggt ggtgaacacg gtgggctcag agaatccgga tgaagccggg cgttacagca 26820
tggatgtgga gtacggtcag tacagtgtca tcctgcaggt tgacggtttt ccaccatcgc 26880
acgccgggac catcaccgtg tatgaagatt cacaaccggg gacgctgaat gattttctct 26940
gtgccatgac ggaggatgat gcccggccgg aggtgctgcg tcgtcttgaa ctgatggtgg 27000
aagaggtggc gcgtaacgcg tccgtggtgg cacagagtac ggcagacgcg aagaaatcag 27060
ccggcgatgc cagtgcatca gctgctcagg tcgcggccct tgtgactgat gcaactgact 27120
cagcacgcgc cgccagcacg tccgccggac aggctgcatc gtcagctcag gaagcgtcct 27180
ccggcgcaga agcggcatca gcaaaggcca ctgaagcgga aaaaagtgcc gcagccgcag 27240
agtcctcaaa aaacgcggcg gccaccagtg ccggtgcggc gaaaacgtca gaaacgaatg 27300
ctgcagcgtc acaacaatca gccgccacgt ctgcctccac cgcggccacg aaagcgtcag 27360
aggccgccac ttcagcacga gatgcggtgg cctcaaaaga ggcagcaaaa tcatcagaaa 27420
cgaacgcatc atcaagtgcc ggtcgtgcag cttcctcggc aacggcggca gaaaattctg 27480
ccagggcggc aaaaacgtcc gagacgaatg ccaggtcatc tgaaacagca gcggaacgga 27540
gcgcctctgc cgcggcagac gcaaaaacag cggcggcggg gagtgcgtca acggcatcca 27600
cgaaggcgac agaggctgcg ggaagtgcgg tatcagcatc gcagagcaaa agtgcggcag 27660
aagcggcggc aatacgtgca gaaaattcgg caaaacgtgc agaagatata gcttcagctg 27720
tcgcgcttga ggatgcggac acaacgagaa aggggatagt gcagctcagc agtgcaacca 27780
acagcacgtc tgaaacgctt gctgcaacgc caaaggcggt taaggtggta atggatgaaa 27840
cgaacagaaa agcccactgg acagtccggc actgaccgga acgccaacag caccaaccgc 27900
gctcagggga acaaacaata cccagattgc gaacaccgct tttgtactgg ccgcgattgc 27960
agatgttatc gacgcgtcac ctgacgcact gaatacgctg aatgaactgg ccgcagcgct 28020
cgggaatgat ccagattttg ctaccaccat gactaacgcg cttgcgggta aacaaccgaa 28080
gaatgcgaca ctgacggcgc tggcagggct ttccacggcg aaaaataaat taccgtattt 28140
tgcggaaaat gatgccgcca gcctgactga actgactcag gttggcaggg atattctggc 28200
aaaaaattcc gttgcagatg ttcttgaata ccttggggcc ggtgagaatt ctaagcggag 28260
atcgcctagt gattttaaac tattgctggc agcattcttg agtccaatat aaaagtattg 28320
tgtacctttt gctgggtcag gttgttcttt aggaggagta aaaggatcaa atgcactaaa 28380

cgaaactgaa acaagcgatc gaaaatatcc ctttgggatt cttgactcga taagtctatt 28440

attttcagag aaaaaatatt cattgttttc tgggttggtg attgcaccaa tcattccatt 28500

caaaattgtt gttttaccac acccattccg cccgataaaa gcatgaatgt tcgtgctggg 28560

catagaatta accgtcacct caaaaggtat agttaaatca ctgaatccgg gagcactttt 28620

tctattaaat gaaaagtgga aatctgacaa ttctggcaaa ccatttaaca cacgtgcgaa 28680

ctgtccatga atttctgaaa gagttacccc tctaagtaat gaggtgttaa ggacgctttc 28740

attttcaatg tcggctaatc gatttggcca tactactaaa tcctgaatag ctttaagaag 28800

gttatgttta aaaccatcgc ttaatttgct gagattaaca tagtagtcaa tgctttcacc 28860

taaggaaaaa aacatttcag ggagttgact gaatttttta tctattaatg aataagtgct 28920

tacttcttct tttgacctta caaaaccaat tttaacattt ccgatatcgc atttttcacc 28980

atgctcatca aagacagtaa gataaaacat tgtaacaaag gaatagtcat tccaaccatc 29040

tgctcgtagg aatgccttat ttttttctac tgcaggaata tacccgcctc tttcaataac 29100

actaaactcc aacatatagt aacccttaat tttattaaaa taaccgcaat ttatttggcg 29160

gcaacacagg atctctcttt taagttactc tctattacat acgttttcca tctaaaaatt 29220

agtagtattg aacttaacgg ggcatcgtat tgtagttttc catatttagc tttctgcttc 29280

cttttggata acccactgtt attcatgttg catggtgcac tgtttatacc aacgatatag 29340

tctattaatg catatatagt atcgccgaac gattagctct tcaggcttct gaagaagcgt 29400

ttcaagtact aataagccga tagatagcca cggacttcgt agccattttt cataagtgtt 29460

aacttccgct cctcgctcat aacagacatt cactacagtt atggcggaaa ggtatgcatg 29520

ctgggtgtgg ggaagtcgtg aaagaaaaga agtcagctgc gtcgtttgac atcactgcta 29580

tcttcttact ggttatgcag gtcgtagtgg gtggcacaca aagctttgca ctggattgcg 29640

aggctttgtg cttctctgga gtgcgacagg tttgatgaca aaaaattagc gcaagaagac 29700

aaaaatcacc ttgcgctaat gctctgttac aggtcactaa taccatctaa gtagttgatt 29760

catagtgact gcatatgttg tgttttacag tattatgtag tctgtttttt atgcaaaatc 29820

taatttaata tattgatatt tatatcattt tacgtttctc gttca 29865

<210> 11
<211> 2244
<212> DNA
<213> Escherichia coli

<220>
<221> source
<222> 1..2244
<223> /mol_type="DNA"
      /organism="Escherichia coli"

<400> 11
atggcgcgtt ccaaaactgc tcagccaaaa cactcactgc gtaaaatcgc agttgtagta 60

gccacagcgg ttagcggcat gtctgtttat gcacaggcag cggttgaacc gaaagaagac 120

actatcaccg ttaccgctgc acctgcgccg caagaaagcg catgggggcc tgctgcaact 180
attgcggcgc gacagtctgc taccggcact aaaaccgata cgccgattca aaaagtgcca 240
cagtctattt ctgttgtgac cgccgaagag atggcgctgc atcagccgaa gtcggtaaaa 300
gaagcgctta gctacacgcc gggtgtctct gttggtacgc gtggcgcatc caacacctat 360
gaccacctga tcattcgcgg ctttgcggca gaaggccaaa gccagaataa ctatctgaat 420
ggcctgaagt tgcagggcaa cttctataac gatgcggtca ttgacccgta tatgctggaa 480
cgcgctgaaa ttatgcgtgg cccggtttcc gtgctttacg gtaaaagcag tcctggcggc 540
ctgttgaata tggtcagcaa gcgtccgacc accgaaccgc tgaaagaagt tcagtttaaa 600
gccggtactg acagcctgtt ccagactggt tttgacttta gcgattcgtt ggatgatgac 660
ggtgtttact cttatcgcct gaccggtctt gcgcgttctg ccaatgccca gcagaaaggg 720
tcagaagagc agcgttatgc tattgcaccg gcgttcacct ggcgtccgga tgataaaacc 780
aattttacct tcctttctta cttccagaac gagccggaaa ccggttatta cggctggttg 840
ccgaaagagg gaaccgttga gccgctgccg aacggtaagc gtctgccgac agactttaat 900
gaaggggcga agaacaacac ctattctcgt aatgagaaga tggtcggcta cagcttcgat 960
cacgaattta acgacacctt tactgtgcgt cagaacctgc gctttgctga aaacaaaacc 1020
tcgcaaaaca gcgtttatgg ttacggcgtc tgctccgatc cggcgaatgc ttacagcaaa 1080
cagtgtgcgg cattagcgcc agcggataaa ggccattatc tggcacgtaa atacgtcgtt 1140
gatgatgaga agctgcaaaa cttctccgtt gatacccagt tgcagagcaa gtttgccact 1200
ggcgatatcg accacaccct gctgaccggt gtcgacttta tgcgtatgcg taatgacatc 1260
aacgcctggt ttggttacga cgactctgtg ccactgctca atctgtacaa tccggtgaat 1320
accgatttcg acttcaatgc caaagatccg gcaaactccg gcccttaccg cattctgaat 1380
aaacagaaac aaacgggcgt ttatgttcag gatcaggcgc agtgggataa agtgctggtc 1440
accctaggcg gtcgttatga ctgggcagat caagaatctc ttaaccgcgt tgccgggacg 1500
accgataaac gtgatgacaa acagtttacc tggcgtggtg gtgttaacta cctgtttgat 1560
aatggtgtaa caccttactt cagctatagc gaatcgtttg aaccttcttc gcaagttggg 1620
aaggatggta atattttcgc accgtctaaa ggtaagcagt atgaagtcgg cgtgaaatat 1680
gtaccggaag atcgtccgat tgtagttact ggtgccgtgt ataatctcac taaaaccaac 1740
aacctgatgg cggaccctga gggttccttc ttctcggttg aaggtggcga gatccgcgca 1800
cgtggcgtag aaatcgaagc gaaagcggcg ctgtcggcga gtgttaacgt agtcggttct 1860
tatacttaca ccgatgcgga atacaccacc gatactacct ataaaggcaa tacgcctgca 1920
caggtgccaa aacacatggc ttcgttgtgg gctgactaca ccttctttga cggtccgctt 1980
tcaggtctga cgctgggcac cggtggtcgt tatactggct ccagttatgg tgatccggct 2040
aactccttta aagtgggaag ttatacggtc gtggatgcgt tagtacgtta tgatctggcg 2100
cgagtcggca tggctggctc caacgtggcg ctgcatgtta acaacctgtt cgatcgtgaa 2160

tacgtcgcca gctgctttaa cacttatggc tgcttctggg gcgcagaacg tcaggtcgtt    2220

gcaaccgcaa ccttccgttt ctaa                                          2244

<210> 12
<211> 1149
<212> DNA
<213> Escherichia coli

<220>
<221> source
<222> 1..1149
<223> /mol_type="DNA"
      /organism="Escherichia coli"

<400> 12
atgagtctga aagaaaaaac acaatctctg tttgccaacg catttggcta ccctgccact      60

cacaccattc aggcgcctgg ccgcgtgaat ttgattggtg aacacaccga ctacaacgac     120

ggtttcgttc tgccctgcgc gattgattat caaaccgtga tcagttgtgc accacgcgat     180

gaccgtaaag ttcgcgtgat ggcagccgat tatgaaaatc agctcgacga gttttccctc     240

gatgcgccca ttgtcgcaca tgaaaactat caatgggcta actacgttcg tggcgtggtg     300

aaacatctgc aactgcgtaa caacagcttc ggcggcgtgg acatggtgat cagcggcaat     360

gtgccgcagg gtgccgggtt aagttcttcc gcttcactgg aagtcgcggt cggaaccgta     420

ttgcagcagc tttatcatct gccgctggac ggcgcacaaa tcgcgcttaa cggtcaggaa     480

gcagaaaacc agtttgtagg ctgtaactgc gggatcatgg atcagctaat ttccgcgctc     540

ggcaagaaag atcatgcctt gctgatcgat tgccgctcac tggggaccaa agcagtttcc     600

atgcccaaag gtgtggctgt cgtcatcatc aacagtaact tcaaacgtac cctggttggc     660

agcgaataca acacccgtcg tgaacagtgc gaaaccggtg cgcgtttctt ccagcagcca     720

gccctgcgtg atgtcaccat tgaagagttc aacgctgttg cgcatgaact ggacccgatc     780

gtggcaaaac gcgtgcgtca tatactgact gaaaacgccc gcaccgttga agctgccagc     840

gcgctggagc aaggcgacct gaaacgtatg ggcgagttga tggcggagtc tcatgcctct     900

atgcgcgatg atttcgaaat caccgtgccg caaattgaca ctctggtaga aatcgtcaaa     960

gctgtgattg gcgacaaagg tggcgtacgc atgaccggcg gcggatttgg cggctgtatc    1020

gtcgcgctga tcccggaaga gctggtgcct gccgtacagc aagctgtcgc tgaacaatat    1080

gaagcaaaaa caggtattaa agagactttt tacgtttgta aaccatcaca aggagcagga    1140

cagtgctga                                                           1149

<210> 13
<211> 1701
<212> DNA
<213> Escherichia coli

<220>
<221> source
<222> 1..1701
<223> /mol_type="DNA"
      /organism="Escherichia coli"

<400> 13
atggcgattg caattggcct cgattttggc agtgattctg tgcgagcttt ggcggtggac 60

tgcgctaccg gtgaagagat cgccaccagc gtagagtggt atccccgttg gcagaaaggg 120

caattttgtg atgccccgaa taaccagttc cgtcatcatc cgcgtgacta cattgagtca 180

atggaagcgg cactgaaaac cgtgcttgca gagcttagcg tcgaacagcg cgcagctgtg 240

gtcgggattg gcgttgacag taccggctcg acgcccgcac cgattgatgc cgacggaaac 300

gtgctggcgc tgcgcccgga gtttgccgaa aacccgaacg cgatgttcgt attgtggaaa 360

gaccacactg cggttgaaga agcggaagag attacccgtt tgtgccacgc gccgggcaac 420

gttgactact cccgctacat tggtggtatt tattccagcg aatggttctg ggcaaaaatc 480

ctgcatgtga ctcgccagga cagcgccgtg gcgcaatctg ccgcatcgtg gattgagctg 540

tgcgactggg tgccagctct gctttccggt accacccgcc cgcaggatat tcgtcgcgga 600

cgttgcagcg ccgggcataa atctctgtgg cacgaaagct ggggcggcct gccgccagcc 660

agtttctttg atgagctgga cccgatcctc aatcgccatt tgccttcccc gctgttcact 720

gacacttgga ctgccgatat tccggtgggc accttatgcc cggaatgggc gcagcgtctc 780

ggcctgcctg aaagcgtggt gatttccggc ggcgcgtttg actgccatat gggcgcagtt 840

ggcgcaggcg cacagcctaa cgcactggta aaagttatcg gtacttccac ctgcgacatt 900

ctgattgccg acaaacagag cgttggcgag cgggcagtta aaggtatttg cggtcaggtt 960

gatggcagcg tggtgcctgg atttatcggt ctggaagcag gccaatcggc gtttggtgat 1020

atctacgcct ggtttggtcg cgtactcggc tggccgctgg aacagcttgc cgcccagcat 1080

ccggaactga aaacgcaaat caacgccagc cagaaacaac tgcttccggc gctgaccgaa 1140

gcatgggcca aaaatccgtc tctggatcac ctgccggtgg tgctcgactg gtttaacggc 1200

cgccgcacac cgaacgctaa ccaacgcctg aaaggggtga ttaccgatct taacctcgct 1260

accgacgctc cgctgctgtt cggcggtttg attgctgcca ccgcctttgg cgcacgcgca 1320

atcatggagt gctttaccga tcaggggatc gccgttaata acgtgatggc actgggcggc 1380

atcgcgcgga aaaaccaggt cattatgcag gcctgctgcg acgtgctgaa tcgcccgctg 1440

caaattgttg cctctgacca gtgctgtgcg ctcggtgcgg cgatttttgc tgccgtcgcc 1500

gcgaaagtgc acgcagacat cccatcagct cagcaaaaaa tggccagtgc ggtagagaaa 1560

accctgcaac cgtgcagcga gcaggcacaa cgctttgaac agctttatcg ccgctatcag 1620

caatgggcga tgagcgccga acaacactat cttccaactt ccgccccggc acaggctgcc 1680

caggccgttg cgactctata a 1701

<210> 14
<211> 1503
<212> DNA
<213> Escherichia coli

<220>
<221> source

<222> 1..1503
<223> /mol_type="DNA"
      /organism="Escherichia coli"

<400> 14
atgacgattt ttgataatta tgaagtgtgg tttgtcattg gcagccagca tctgtatggc      60

ccggaaaccc tgcgtcaggt cacccaacat gccgagcacg tcgttaatgc gctgaatacg     120

gaagcgaaac tgccctgcaa actggtgttg aaaccgctgg gcaccacgcc ggatgaaatc     180

accgctattt gccgcgacgc gaattacgac gatcgttgcg ctggtctggt ggtgtggctg     240

cacaccttct ccccggccaa aatgtggatc aacggcctga ccatgctcaa caaaccgttg     300

ctgcaattcc acacccagtt caacgcggcg ctgccgtggg acagtatcga tatggacttt     360

atgaacctga accagactgc acatggcggt cgcgagttcg gcttcattgg cgcgcgtatg     420

cgtcagcaac atgccgtggt taccggtcac tggcaggata aacaagccca tgagcgtatc     480

ggctcctgga tgcgtcaggc ggtctctaaa caggataccc gtcatctgaa agtctgccga     540

tttggcgata acatgcgtga agtggcggtc accgatggcg ataaagttgc cgcacagatc     600

aagttcggtt tctccgtcaa tacctgggcg gttggcgatc tggtgcaggt ggtgaactcc     660

atcagcgacg gcgatgttaa cgcgctggtc gatgagtacg aaagctgcta caccatgacg     720

cctgccacac aaatccacgg caaaaaacga cagaacgtgc tggaagcggc gcgtattgag     780

ctggggatga agcgtttcct ggaacaaggt ggcttccacg cgttcaccac cacctttgaa     840

gatttgcacg gtctgaaaca gcttcctggt ctggccgtac agcgtctgat gcagcagggt     900

tacggctttg cgggcgaagg cgactggaaa actgccgccc tgcttcgcat catgaaggtg     960

atgtcaaccg gtctgcaggg cggcacctcc tttatggagg actacaccta tcacttcgag    1020

aaaggtaatg acctggtgct cggctcccat atgctggaag tctgcccgtc gatcgccgca    1080

gaagagaaac cgatcctcga cgttcagcat ctcggtattg gtggtaagga cgatcctgcc    1140

cgcctgatct tcaataccca aaccggccca gcgattgtcg ccagcttgat tgatctcggc    1200

gatcgttacc gtctactggt taactgcatc gacacggtga aaacaccgca ctccctgccg    1260

aaactgccgg tggcgaatgc gctgtggaaa gcgcaaccgg atctgccaac tgcttccgaa    1320

gcgtggatcc tcgctggtgg cgcgcaccat accgtcttca gccatgcact gaacctcaac    1380

gatatgcgcc aattcgccga gatgcacgac attgaaatca cggtgattga taacgacaca    1440

cgcctgccag cgtttaaaga cgcgctgcgc tggaacgaag tgtattacgg gtttcgtcgc    1500

taa                                                                  1503


<210> 15
<211> 2355
<212> DNA
<213> Escherichia coli

<220>
<221> source
<222> 1..2355
<223> /mol_type="DNA"
      /organism="Escherichia coli"

<400> 15
atgaatcctg agcgttctga acgcattgaa atccccgtat tgccgctgcg cgatgtggtg 60
gtttatccgc acatggtcat ccccttattt gtcgggcggg aaaaatctat ccgttgtctg 120
gaagcggcga tggaccatga taaaaaaatt atgctggtcg cgcagaaaga agcttcaacg 180
gatgagccgg gtgtaaacga tcttttcacc gtcgggaccg tggcctctat attgcagatg 240
ctgaaactgc ctgacggcac cgtcaaagtg ctggtcgagg ggttacagcg cgcgcgtatt 300
tctgcgctct ctgacaatgg cgaacacttt tctgcgaagg cggagtatct ggagtcgccg 360
accattgatg agcgggaaca ggaagtgctg gtgcgtactg caatcagcca gttcgaaggc 420
tacatcaagc tgaacaaaaa aatcccacca gaagtgctga cgtcgctgaa tagcatcgac 480
gatccggcgc gtctggcgga taccattgct gcacatatgc cgctgaaact ggctgacaaa 540
cagtctgttc tggagatgtc cgacgttaac gaacgtctgg aatatctgat ggcaatgatg 600
gaatcggaaa tcgatctgct gcaggttgag aaacgcattc gcaaccgcgt taaaaagcag 660
atggagaaat cccagcgtga gtactatctg aacgagcaaa tgaaagctat tcagaaagaa 720
ctcggtgaaa tggacgacgc gccggacgaa aacgaagccc tgaagcgcaa aatcgacgcg 780
gcgaagatgc cgaaagaggc aaaagagaaa gcggaagcag agttgcagaa gctgaaaatg 840
atgtctccga tgtcggcaga agcgaccgta gtgcgtggtt atatcgactg gatggtacag 900
gtgccgtgga atgcgcgtag caaggtcaaa aaagacctgc gtcaggcgca ggaaatcctt 960
gataccgacc attatggtct ggagcgcgtg aaagatcgaa tccttgagta tcttgcggtt 1020
caaagccgtg tcaacaaaat caagggaccg atcctctgcc tggtagggcc gccgggggta 1080
ggtaaaacct ctcttggtca gtccattgcc aaagccaccg ggcgtaaata tgtccgtatg 1140
gcgctgggcg gcgtgcgtga tgaagcggaa atccgtggtc accgccgtac ttacatcggt 1200
tctatgccgg gtaaactgat ccagaaaatg gcgaaagtgg gcgtgaaaaa cccgctgttc 1260
ctgctcgatg agatcgacaa aatgtcttct gacatgcgtg gcgatccggc ctctgcactg 1320
cttgaagtgc tggatccaga gcagaacgta gcgttcagcg accactacct ggaagtggat 1380
tacgatctca gcgacgtgat gtttgtcgcg acgtcgaact ccatgaacat tccggcaccg 1440
ctgctggatc gtatggaagt gattcgcctc tccggttata ccgaagatga aaaactgaac 1500
atcgccaaac gtcacctgct gccgaagcag attgaacgta atgcactgaa aaaaggtgag 1560
ctgaccgtcg acgatagcgc cattatcggc attattcgtt actacacccg tgaggcgggc 1620
gtgcgtggtc tggagcgtga aatctccaaa ctgtgtcgca aagcggttaa gcagttactg 1680
ctcgataagt cattaaaaca tatcgaaatt aacggcgata acctgcatga ctatctcggt 1740
gttcagcgtt tcgactatgg tcgcgcggat aacgaaaacc gtgtcggtca ggtaaccggt 1800
ctggcgtgga cggaagtggg cggtgacttg ctgaccattg aaaccgcatg tgttccgggt 1860
aaaggcaaac tgacctatac cggttcgctc ggcgaagtga tgcaggagtc cattcaggcg 1920
gcgttaacgg tggttcgtgc gcgtgcggaa aaactgggga tcaaccctga tttttacgaa 1980

aaacgtgaca tccacgtcca cgtaccggaa ggtgcgacgc cgaaagatgg tccgagtgcc 2040

ggtattgcta tgtgcaccgc gctggtttct tgcctgaccg gtaacccggt tcgtgccgat 2100

gtggcaatga ccggtgagat cactctgcgt ggtcaggtac tgccgatcgg tggtttgaaa 2160

gaaaaactcc tggcagcgca tcgcggcggg attaaaacag tgctaattcc gttcgaaaat 2220

aaacgcgatc tggaagagat tcctgacaac gtaattgccg atctggacat tcatcctgtg 2280

aagcgcattg aggaagttct gactctggcg ctgcaaaatg aaccgtctgg tatgcaggtt 2340

gtgactgcaa aatag 2355

<210> 16
<211> 954
<212> DNA
<213> Escherichia coli

<220>
<221> source
<222> 1..954
<223> /mol_type="DNA"
      /organism="Escherichia coli"

<400> 16
atgcgggcga aacttctggg aatagtcctg acaaccccta ttgcgatcag ctcttttgct 60

tctaccgaga ctttatcgtt tactcctgac aacataaatg cggacattag tcttggaact 120

ctgagcggaa aaacaaaaga gcgtgtttat ctagccgaag aaggaggccg aaaagtcagt 180

caactcgact ggaaattcaa taacgctgca attattaaag gtgcaattaa ttgggatttg 240

atgccccaga tatctatcgg ggctgctggc tggacaactc tcggcagccg aggtggcaat 300

atggtcgatc aggactggat ggattccagt aaccccggaa cctggacgga tgaaagtaga 360

caccctgata cacaactcaa ttatgccaac gaatttgatc tgaatatcaa aggctggctc 420

ctcaacgaac ccaattaccg cctgggactc atggccggat atcaggaaag ccgttatagc 480

tttacagcca gaggtggttc ctatatctac agttctgagg agggattcag agatgatatc 540

ggctccttcc cgaatggaga aagagcaatc ggctacaaac aacgttttaa aatgccctac 600

attggcttga ctggaagtta tcgttatgaa gattttgaac tcggtggcac atttaaatac 660

agcggctggg tggaatcatc tgataacgat gaacactatg acccgggaaa aagaatcact 720

tatcgcagta aggtcaaaga ccaaaattac tattctgttg cagtcaatgc aggttattac 780

gtcacaccta acgcaaaagt ttatgttgaa ggcgcatgga atcgggttac gaataaaaaa 840

ggtaatactt cactttatga tcacaataat aacacttcag actacagcaa aaatggagca 900

ggtatagaaa actataactt catcactact gctggtctta agtacacatt ttaa 954

<210> 17
<211> 624
<212> DNA
<213> Escherichia coli

<220>
<221> source
<222> 1..624

<223> /mol_type="DNA"
      /organism="Escherichia coli"

<400> 17
atgtcaacga ttattatgga tttatgtagt tacacccgac taggtttaac cgggtatctg      60

ttgagtagag gggttaaaaa aagagaaatc aacgacattg aaaccgttga tgaccttgcc     120

atagcttgtg attcacagcg cccttcagtg gtgtttatta atgaggactg tttcatccac     180

gatgcttcta acagtcagcg tatcaagctc atcattaatc aacatcccaa tacgttattt     240

atcgttttta tggcaattgc caatgttcat tttgatgaat atctattggt cagaaaaaat     300

ttattgatca gttctaaatc gattaaaccg gaatctctcg acgatatcct tggcgatatt     360

ctgaaaaaag agacaacgat aacctcgttt ttaaatatgc cgacgttatc attgagccga     420

accgaatcga gtatgttgcg aatgtggatg gcaggtcagg gaaccattca aatctctgac     480

caaatgaata tcaaagccaa gaccgtttca tcgcataaag gtaatattaa acgtaagatc     540

aaaacgcata ataaacaggt tatctaccat gtcgtccgac tgacggataa tgtgactaat     600

ggtatttttg tcaacatgcg ctaa                                            624


<210> 18
<211> 3567
<212> DNA
<213> Escherichia coli

<220>
<221> source
<222> 1..3567
<223> /mol_type="DNA"
      /organism="Escherichia coli"

<400> 18
atgttatggg ccttaaatat ttggacaggc ccgcacagca atggattaat aacaatgatg      60

aataaatcca attttgaatt cctgaagggc gtcaacgact tcacttatgc catcgcctgt     120

gcggcggaaa ataactaccc ggatgatccc aacacgacgc tgattaaaat gcgtatgttt     180

ggcgaagcca cagcgaaaca tcttggtctg ttactcaaca tccccccttg tgagaatcaa     240

cacgatctcc tgcgtgaact cggcaaaatc gcctttgttg atgacaacat cctctctgta     300

tttcacaaat tacgccgcat tggtaaccag gcggtgcacg aatatcataa cgatctcaac     360

gatgcccaga tgtgcctgcg actcgggttc cgcctggctg tctggtacta ccgtctggtc     420

actaaagatt atgacttccc ggtgccggtg tttgtgttgc cggaacgtgg tgaaaacctc     480

tatcaccagg aagtgctgac gctaaaacaa cagcttgaac agcaggtgcg agaaaaagcg     540

cagactcagg cagaagtcga agcgcaacag cagaagctgg ttgccctgaa cggctatatc     600

gccattctgg aaggcaaaca gcaggaaacc gaagcgcaaa cccaggctcg ccttgcggca     660

ctggaagcac agctcgccga gaagaacgcg gaactggcaa aacagaccga acaggaacgt     720

aaggcttacc acaaagaaat taccgatcag gccatcaagc gcacactcaa ccttagcgaa     780

gaagagagtc gcttcctgat tgatgcgcaa ctgcgtaaag caggctggca ggccgacagc     840

aaaaccctgc gcttctccaa aggcgcacgt ccggaacccg gcgtcaataa agccattgcc     900

gaatggccga ccggaaaaga tgaaacgggt aatcagggct ttgcggatta tgtgctgttt 960
gtcggcctca aacccatcgc ggtggtagag gcgaaacgta acaatatcga cgttcccgcc 1020
aggctcaatg agtcgtatcg ctacagtaaa tgtttcgata atggcttcct gcgggaaacc 1080
ttgcttgagc actactcacc ggatgaagtg catgaagcag tgccagagta tgaaaccagc 1140
tggcaggaca ccagcggcaa acaacggttt aaaatcccct tctgctactc gaccaacggg 1200
cgcgaatacc gcgcaacaat gaagaccaaa agcggcatct ggtatcgcga cgtgcgtgat 1260
acccgcaata tgtcgaaagc cttacccgag tggcaccgcc cggaagagct gctggaaatg 1320
ctcggcagcg aaccgcaaaa acagaatcag tggtttgccg ataaccctgg catgagcgag 1380
ctgggcctgc gttattatca ggaagatgcc gtccgcgcgg ttgaaaaggc aatcgtcaag 1440
gggcaacaag agatcctgct ggcgatggcg accggtaccg gtaaaacccg tacggcaatc 1500
gccatgatgt tccgcctgat ccagtcccag cgttttaaac gcattctctt ccttgtcgac 1560
cgccgttctc ttggcgaaca ggcgctgggc gcgtttgaag atacgcgtat taacggcgac 1620
accttcaaca gcattttcga cattaaaggg ctgacggata aattcccgga agacagcacc 1680
aaaattcacg ttgccaccgt acagtcgctg gtgaaacgca ccctgcaatc agatgaaccg 1740
atgccggtgg cccgttacga ctgtatcgtc gttgacgaag cgcatcgcgg ctatattctc 1800
gataaagagc agaccgaagg cgaactgcag ttccgcagcc agctggatta cgtctctgcc 1860
taccgtcgca ttctcgatca cttcgatgcg gtaaaaatcg ctctcaccgc caccccggcg 1920
ctacatactg tgcagatttt cggcgagccg gtttaccgtt atacctaccg taccgcggtt 1980
atcgacggtt ttctgatcga ccaggatccg cctattcaga tcatcacccg caacgcgcag 2040
gagggggttt atctctccaa aggcgagcag gtagagcgca tcagcccgca gggagaagtg 2100
atcaatgaca ccctggaaga cgatcaggat tttgaagtcg ccgactttaa ccgtggcctg 2160
gtgatcccgg cgtttaaccg cgccgtctgt aacgaactca ccaattatct tgacccgacc 2220
ggatcgcaaa aaacgctggt cttctgcgtc accaatgccc atgccgatat ggtggtggaa 2280
gagctgcgtg ccgcgttcaa gaaaaagtat ccgcaactgg agcacgacgc gatcatcaag 2340
atcaccggtg atgccgataa agacgcgcgc aaagtgcaga ccatgatcac ccgcttcaat 2400
aaagagcggc tgcccaatat cgtggtaacc gtcgacctgc tgacgaccgg cgtcgatatt 2460
ccgtcgatct gtaatatcgt gttcctgcgt aaagtacgca gccgcattct gtacgaacag 2520
atgaaaggcc gcgccacgcg cttatgcccg gaggtgaata aaaccagctt taagattttt 2580
gactgtgtcg atatctacag cacgctggag agcgtcgaca ccatgcgtcc ggtggtggtg 2640
cgcccgaagg tggaactgca aacgctggtc aatgaaatta ccgattcaga aacctataaa 2700
atcaccgaag cggatggccg cagttttgcc gagcacagcc atgaacaact ggtggcgaag 2760
ctccagcgta tcatcggtct ggccacgttt aaccgtgacc gcagcgaaac gatagataaa 2820
caggtgcgtc gtctggatga gctatgccag gacgcggcgg gcgtgaactt taacggcttc 2880
gcctcgcgcc tgcgggaaaa agggccgcac tggagcgccg aagtctttaa caaactgcct 2940

ggctttatcg cccgtctgga aaagctgaaa acggacatca acaacctgaa tgatgcgccg 3000

atcttcctcg atatcgacga tgaagtggtg agtgtaaaat cgctgtacgg tgattacgac 3060

acgccgcagg atttcctcga agcctttgac tcgctggtgc aacgttcccc gaacgcgcaa 3120

ccggcattgc aggcagttat taatcgcccg cgcgatctca cccgtaaagg gctggtcgag 3180

ctacaggagt ggtttgaccg ccagcacttt gaggaatctt ccctgcgcaa agcatggaaa 3240

gagacgcgca atgaagatat cgccgcccgg ctgattggtc atattcgccg cgctgcggtg 3300

ggcgatgcgc tgaaaccgtt tgaggaacgt gtcgatcacg cgctgacgcg cattaagggc 3360

gaaaacgact ggagcagcga gcaattaagc tggctcgatc gtttagcgca ggcgctgaaa 3420

gagaaagtgg tgctcgacga cgatgtcttc aaaaccggca acttccaccg tcgcggcggg 3480

aaggcgatgc tgcaaagaac ctttgacgat aatctcgata ccctgctggg caaattcagc 3540

gattatatct gggacgagct ggcctga 3567

<210> 19
<211> 915
<212> DNA
<213> Escherichia coli

<220>
<221> source
<222> 1..915
<223> /mol_type="DNA"
/organism="Escherichia coli"

<400> 19
atgacggttc ctacctatga caaatttatt gaacctgttc tgcgttatct ggcaacaaaa 60

ccggaaggtg cagccgcgcg tgatgttcat gaggctgccg cggatgcatt aggactggat 120

gacagccagc gagcgaaagt cattaccagc ggacaacttg tttataaaaa tcgtgcaggc 180

tgggcgcatg accgtttaaa acgtgccggg ttgtcgcaaa gtttgtcgcg tggcaaatgg 240

tgcctgactc ctgcgggttt tgactgggtt gcgtctcatc cccagccaat gacggagcag 300

gagacgaacc atctggcctt cgcttttgtg aatgtcaaac ttaagtcacg gccggatgcc 360

gtcgatttag atccgaaagc cgactctccc gatcatgaag aacttgcaaa gagcagcccg 420

gacgatcggt tagatcaggc gctaaaagag cttcgtgatg cggtggctga tgaggttctg 480

gaaaacttat tgcaggtttc tccttcgcgc tttgaagtca ttgttctgga tgttttgcat 540

cgcctggggt atggcggcca ccgtgatgat ttgcagcgtg ttggcggtac tggagatggt 600

ggcatcgatg gtgtgatatc gcttgataaa cttggcctgg agaaagttta tgttcaggca 660

aaacgttggc agaatactgt aggcaggcca gaattacagg cattttacgg cgcactggct 720

gggcaaaaag cgaaacgtgg ggtgtttatt accacttctg gatttacttc tcaggcgcgt 780

gactttgccc aatccgtcga gggtatggtg ttggttgatg gggaacgcct ggtgcactta 840

atgatcgaaa acgaagtagg ggtttcttca cgtttgttga aggtgccgaa actggatatg 900

gactattttg agtga 915

<210> 20
<211> 708
<212> DNA
<213> Escherichia coli

<220>
<221> source
<222> 1..708
<223> /mol_type="DNA"
      /organism="Escherichia coli"

<400> 20
atgtaccgtt atttgtctat tgctgcggtg gtactgagcg cagcattttc cggcccggcg      60

ttggccgaag gtatcaatag tttttctcag gcgaaagccg cggcggtaaa agtccacgct     120

gacgcgcccg gtacgtttta ttgcggatgt aaaattaact ggcagggcaa aaaaggcgtt     180

gttgatctgc aatcgtgcgg ctatcaggtg cgcaaaaatg aaaaccgcgc cagccgcgta     240

gagtgggaac atgtcgttcc cgcctggcag ttcggtcacc agcgccagtg ctggcaggac     300

ggtggacgta aaaactgcgc taaagatccg gtctatcgca agatggaaag cgatatgcat     360

aacctgcagc cgtcagtcgg tgaggtgaat ggcgatcgcg gcaactttat gtacagccag     420

tggaatggcg gtgaaggcca gtacggtcaa tgcgccatga aggtcgattt caaagaaaaa     480

gctgccgaac caccagcgcg tgcacgcggt gccattgcgc gcacctactt ctatatgcgc     540

gaccaataca acctgacact ctctcgccag caaacgcagc tgttcaacgc atggaacaag     600

atgtatccgg ttaccgactg ggagtgcgag cgcgatgaac gcatcgcgaa ggtgcagggc     660

aatcataacc cgtatgtgca acgcgcttgc caggcgcgaa agagctaa                  708


<210> 21
<211> 1062
<212> DNA
<213> Escherichia coli

<220>
<221> source
<222> 1..1062
<223> /mol_type="DNA"
      /organism="Escherichia coli"

<400> 21
atggctatcg acgaaaacaa acagaaagcg ttggcggcag cactgggcca gattgagaaa      60

caatttggta aaggctccat catgcgcctg ggtgaagacc gttccatgga tgtggaaacc     120

atctctaccg gttcgctttc actggatatc gcgcttgggg caggtggtct gccgatgggc     180

cgtatcgtcg aaatctacgg accggaatct tccggtaaaa ccacgctgac gctgcaggtg     240

atcgccgcag cgcagcgtga aggtaaaacc tgtgcgttta tcgatgctga acacgcgctg     300

gacccaatct acgcacgtaa actgggcgtc gatatcgaca acctgctgtg ctcccagccg     360

gacaccggcg agcaggcact ggaaatctgt gacgccctgg cgcgttctgg cgcagtagac     420

gttatcgtcg ttgactccgt ggcggcactg acgccgaaag cggaaatcga aggcgaaatc     480

ggcgactctc acatgggcct tgcggcacgt atgatgagcc aggcgatgcg taagctggcg     540

ggtaacctga agcagtccaa cacgctgctg atcttcatca accagatccg tatgaaaatt 600

ggtgtgatgt tcggtaaccc ggaaaccact accggtggta acgcgctgaa attctacgcc 660

tctgttcgtc tcgacatccg tcgtatcggc gcggtgaaag agggcgaaaa cgtggtgggt 720

agcgaaaccc gcgtgaaagt ggtgaagaac aaaatcgctg cgccgtttaa acaggctgaa 780

ttccagatcc tctacggcga aggtatcaac ttctacggcg aactggttga cctgggcgta 840

aaagagaagc tgatcgagaa agcaggcgcg tggtacagct acaaaggtga gaagatcggt 900

cagggtaaag cgaatgcgac tgcctggctg aaagataacc cggaaaccgc gaaagagatc 960

gagaagaaag tacgtgagtt gctgctgagc aacccgaact caacgccgga tttctctgta 1020

gatgatagcg aaggcgtagc agaaactaac gaagattttt aa 1062

<210> 22
<211> 306
<212> DNA
<213> Escherichia coli

<220>
<221> source
<222> 1..306
<223> /mol_type="DNA"
      /organism="Escherichia coli"

<400> 22
atgcagttta aggtttacac ctataaaaga gagagccgtt atcgtctgtt tgtggatgta 60

cagagtgata ttattgacac gcccgggcga cggatggtga tccccctggc cagtgcacgt 120

ctgctgtcag ataaagtctc ccgtgaactt tacccggtgg tgcatatcgg ggatgaaagc 180

tggcgcatga tgaccaccga tatggccagt gtgccggtct ccgttatcgg ggaagaagtg 240

gctgatctca gccaccgcga aaatgacatc aaaaacgcca ttaacctgat gttctgggga 300

atataa 306

<210> 23
<211> 219
<212> DNA
<213> Escherichia coli

<220>
<221> source
<222> 1..219
<223> /mol_type="DNA"
      /organism="Escherichia coli"

<400> 23
atgaagcagc gtattacagt gacagttgac agcgacagct atcagttgct caaggcatat 60

gatgtcaata tctccggtct ggtaagcaca accatgcaga atgaagcccg tcgtctgcgt 120

gccgaacgct ggaaagcgga aaatcaggaa gggatggctg aggtcgcccg gtttattgaa 180

atgaacggct cttttgctga cgagaacagg gactggtga 219

<210> 24
<211> 59
<212> DNA

<213> artificial sequences

<220>
<221> source
<222> 1..59
<223> /mol_type="DNA"
      /note="primers XisgalKstart"
      /organism="artificial sequences"

<400> 24
gggggtaaat cccggcgctc atgacttcgc cttcttccca gaattcctgt tgacaatta      59


<210> 25
<211> 60
<212> DNA
<213> artificial sequences

<220>
<221> source
<222> 1..60
<223> /mol_type="DNA"
      /note="XisgalK stop"
      /organism="artificial sequences"

<400> 25
gttctgatta ttggaaatct tctttgccct ccagtgtgag cagcactgtc ctgctccttg      60


<210> 26
<211> 20
<212> DNA
<213> artificial sequences

<220>
<221> source
<222> 1..20
<223> /mol_type="DNA"
      /note="Xis1 primer"
      /organism="artificial sequences"

<400> 26
gtcttcaagt ggagcatcag                                                  20


<210> 27
<211> 20
<212> DNA
<213> artificial sequences

<220>
<221> source
<222> 1..20
<223> /mol_type="DNA"
      /note="Xis4 primer"
      /organism="artificial sequences"

<400> 27
accaggacta tccgtatgac                                                  20


<210> 28
<211> 35
<212> DNA
<213> artificial sequences

<220>
<221> source

<222> 1..35
<223> /mol_type="DNA"
      /note="Xis2 primer"
      /organism="artificial sequences"

<400> 28
ccaaacggaa cagatgaaga aggcgaagtc atgag                                35


<210> 29
<211> 35
<212> DNA
<213> artificial sequences

<220>
<221> source
<222> 1..35
<223> /mol_type="DNA"
      /note="Xis3 primer"
      /organism="artificial sequences"

<400> 29
gacttcgcct tcttcatctg ttccgtttgg cttcc                                35


<210> 30
<211> 21
<212> DNA
<213> artificial sequences

<220>
<221> source
<222> 1..21
<223> /mol_type="DNA"
      /note="Xis6 primer"
      /organism="artificial sequences"

<400> 30
gtaatggaaa gctggtagtc g                                               21


<210> 31
<211> 20
<212> DNA
<213> artificial sequences

<220>
<221> source
<222> 1..20
<223> /mol_type="DNA"
      /note="Xis5 primer"
      /organism="artificial sequences"

<400> 31
cagccgtaag tcttgatctc                                                 20


<210> 32
<211> 20
<212> DNA
<213> artificial sequences

<220>
<221> source
<222> 1..20
<223> /mol_type="DNA"
      /note="Xis7 primer"
      /organism="artificial sequences"

<400> 32
cagcaggcat gatccaagag                                                    20


<210> 33
<211> 35
<212> DNA
<213> artificial sequences

<220>
<221> source
<222> 1..35
<223> /mol_type="DNA"
      /note="Xis2b primer"
      /organism="artificial sequences"

<400> 33
tttgccctcc agtgtgaaga aggcgaagtc atgag                                   35


<210> 34
<211> 39
<212> DNA
<213> artificial sequences

<220>
<221> source
<222> 1..39
<223> /mol_type="DNA"
      /note="Xis8 primer"
      /organism="artificial sequences"

<400> 34
ctcatgactt cgccttcttc acactggagg gcaaagaag                               39